# EUROPEAN PATENT APPLICATION

(11) **EP 4 056 199 A1**
(43) Date of publication of application: **14.09.2022**
(21) Application number: 21020138.0
(22) Date of filing: 10.03.2021
(51) Int. Cl.: A61K 39/12, A61P 31/14

(54) **OPTIMIZED VACCINE TO ELICIT A T CELL IMMUNITY AGAINST SARS-COV-2**

(71) Applicant: Kosmatopoulos, Kostantinos (Kostas), 75005 Paris (FR); Menez-Jamet, Jeanne, 92120 Montrouge (FR); Kinet, Jean-Pierre, 13100 Aix-en-Provence (FR)
(72) Inventor: KOSMATOPOULOS, Kostantinos, 75005 Paris (FR); MENEZ-JAMET, Jeanne, 92120 Montrouge (FR); KINET, Jean-Pierre, 13100 Aix-en-Provence (FR)
(74) Representative: Santarelli

(57) **Abstract**

The invention pertains to immunogenic compositions designed to elicit both a humoral and a T-cell immunity against SARS-CoV-2. To this aim, the invention provides modified sequences of the S protein and/or the N protein of SARS-CoV-2, in which each of the introduced modifications is likely to increase the affinity of a cryptic epitope presented by one of the major HLA-I molecules (HLA-A1, -A2, -A3, -A24, -B7, - B35 and Cw7) for this HLA-I molecule

## Description

### FIELD OF THE INVENTION

The present invention relates to the field of vaccination. More specifically, it relates to immunogenic compositions designed to elicit both a humoral and a T-cell immunity against SARS-CoV-2, which is responsible for Coronavirus disease 2019 (COVID-19).

### BACKGROUND OF THE INVENTION

Patients infected by SARS-CoV-2 develop a humoral and cellular virus specific immunity. This immunity is stronger in symptomatic than in asymptomatic patients.

Neutralizing IgG antibodies (Ab) recognize the spike antigen (protein S) and particularly the S1 subunit (aa 14-685) containing the Receptor Binding Domain (RBD) (aa 319-541) thus preventing the binding of the virus to the ACE2 receptor. IgG Ab appear one week following symptoms onset, increase during the first three weeks and begin to decrease by 8 weeks. Humoral response seems therefore to be shortlasting (3-4 months) (Poland et al., 2020).

Cellular immunity mediated by CD4+ and CD8+ T cells is predominantly directed against proteins S, M, N and ORF1ab of the virus. CD4 T cell response seems to be stronger than CD8 T cell response. Cellular immunity appears a couple of days following symptom onset. Interestingly the majority of patients (around 80%) with undetectable humoral response have a T cell response (Grifoni et al., 2020; Schwartzkopf et al., 2021). Several immunodominant epitopes have been very recently described but results are very often contradictory among different publications, probably due to the different tests used for their identification. Table 1 shows the described immunodominant epitopes (9 or 10 aa) from the proteins S and N that are detected in two or more tested patients (Ferretti A. et al. 2020; Tarke et al., 2021).

**Table 1: Immunodominant epitopes (9 or 10 aa) from the proteins S and N that are detected in two or more tested patients (Ferretti A. et al. 2020; Tarke et al., 2021)**

| HLA allele | 1^{st} position of the epitope | Sequence | Parent protein | SEQ ID No |
|---|---|---|---|---|
| HLA-A*0201 | 269 | YLQPRTFLL | S | 157 |
| HLA-A*0201 | 222 | LLLDRLNQL | N | 158 |
| HLA-A*2402 | 312 | IYQTSNFRV | S | 159 |
| HLA-A*2402 | 1208 | QYIKWPWYI | S | 160 |
| HLA-A*0301 | 41 | KVFRSSVLH | S | 161 |
| HLA-A*0301 | 378 | KCYGVSPTK | S | 162 |
| HLA-A*0301 | 361 | KTFPPTEPK | N | 163 |
| HLA-B*0702 | 506 | QPYRVVVLSF | S | 164 |
| HLA-B*0702 | 1052 | FPQSAPHGV | S | 165 |
| HLA-B*0702 | 105 | SPRWYFYYL | N | 166 |
| HLA-B*3501 | 687 | VASQSIIAY | S | 167 |
| HLA-B*3501 | 266 | KAYNVTQAF | N | 168 |
| HLA-B*3501 | 79 | SPDDQIGYY | N | 169 |
| HLA-B*3501 | 307 | FAPSASAFF | N | 170 |
| HLA-B*3501 | 325 | TPSGTWLTY | N | 171 |
| HLA-B*3501 | 335 | LPAADLDDF | N | 172 |
| HLA-A*1101 | 361 | KTFPPTEPK | N | 173 |
| HLA-A*1101 | 134 | ATEGALNTPK | N | 174 |
| HLA-A*1101 | 311 | ASAFFGMSR | N | 175 |

It is worthy to note that there is a pre-exiting T cell immunity against SARS-CoV-2 in around 30% of people probably due to previous infections with human coronaviruses. Pre-existing humoral immunity has not been observed (Le Bert et al., 2020; Grifoni et al., 2020; Sette and Crotty 2021).

Little is known about the protective immunity mediated by T cells because the large majority of research studies to date focused on the humoral response. However, T cell immunity should play an important role in eliminating virus as is the case with other viruses including SARS-CoV and SMERS.

Moreover, little is known about the B cell and T cell memory response against SARS-CoV-2 since the follow-up of the COVID-19 is still short. Some studies showed the presence of memory T cells in the majority of patients 6 months after infection (Zuo et al., 2020; Dan et al., 2021).

Three vaccines against SARS-CoV-2 are very largely and successfully being used (AZD 1222, mRNA-1273 and BNT162b2). All three induce both humoral and cellular immunity (Walsh et al., 2020; Sahin et al., 2020; Widge et al., 2021; Ramasamy et al., 2020).

Several variants of the SARS-CoV-2 have recently been identified. Somme of them spread in several countries and co-exist or sometimes replaced the initial strain (or "variant 0", initially identified in Wuhan in December 2019). This is the case of B.1.1.7 (seven mutations on the protein S: N501Y, A570D, P681H, T716I, S982A, D1118H and deletion of 144Y; two mutations of the protein N: D3L and S235F), B1.351 (nine mutations of the protein S: L18F, D80A, D215G, R246I, K417N, E484K, N501Y, D614G and A701V), P1 (10 mutations on the protein S; L18F, T20N, P26S, D138Y, R190S, K417T, E484K, N501Y, H655Y and T1027I; one mutation on protein N P80R), B1.525 (four mutations on the protein S; Q52R, E484K, Q677H, F888L) and B1.429 (CA20c) (three mutations in the protein S: S13I, W152C and L452R). It seems that all of them (except the B1.1.7 and probably the B1.429) partially escape the humoral immunity triggered by the initial strain and by the vaccines. There is still no clear data on the sensibility of these variants to the T cell immunity. Although the T cell, mainly CD8, epitopes of the different antigens of the SARS-CoV-2 have not yet been definitively and completely identified, it is important to note that some of these mutations might alter the sequence and/or the processing of CD8 epitopes presented in Table1 (Table 2).

**Table 2: Mutations of the most frequent variants of SRAS-COV-2 likely to alter the sequence and/or the processing of CD8 immunodominant epitopes of S and N proteins**

| Epitope | Parent protein | P1 | B1.351 | B1.1.7 |
|---|---|---|---|---|
| 506/HLA-B*0702 | S | N501Y | N501Y | N501Y |
| 687/HLA-B*3501 | S | | | P681H |
| 79/HLA-B*3501 | N | P80R | P80R | |
| 222//HLA-A*0201 | N | | T205I | |

Preliminary data that should be confirmed showed that T cells stimulated in patients infected by the initial strain are less reactive against the B1.1.7, P1 and B1.429 variants than against the initial strain. Moreover, T cells stimulated by the vaccines mRNA-1273 and BNT162b2 are less reactive against the B1.1.7, B1.351 and B1.429 variants than against the initial strain (Tarke et al., 2021).

The emergence of immune escape variants is a very important issue of the protective immunity either natural, induced by the virus, or induced by a vaccine. Two approaches could be proposed to solve this problem. The first is to adapt existing vaccines to induce immunity against the new variants. This can be considered only if the frequency of new variants is low and the delay of appearance of new variants is quite long. This does not seem to be the case of SARS-CoV-2. The second approach could be to design a new vaccine combining T cell and B cell protective immunity that could very likely protect against initial strain and all already existing and future variants.

The present invention aims at providing such a vaccine.

### SUMMARY OF THE INVENTION

The present invention pertains to a new vaccine, which is able to induce a humoral immune response against the RBD of the S protein and a T cell immune response against several cryptic epitopes of the S protein and/or the N protein of SARS-CoV-2.

An object of the invention is a modified S protein comprising at least 4 modifications selected amongst L24A, K77Y, T109Y, D138Y, M153T, E169R, Q173Y, I197T, I714Y, I714A, S721Y, E725K, L727Y, S810Y, S813Y, K814Y, L821Y, G838T, Q853Y, G857Y, V915Y, S937Y, V976Y, S982Y, R983Y, V1040T, H1048Y, A1056Y, V1060Y, A1087Y, I1183T, N1192Y, W1212A, M1214R, G1223Y and V1264K, with the proviso that:
- positions 169 and 173 are not simultaneously modified;
- positions 714 and 721 are not simultaneously modified;
- at most one of positions 721, 725 and 727 is modified;
- at most one of positions 810, 813 and 814 is modified;
- at most one of positions 813, 814 and 821 is modified;
- positions 853 and 857 are not simultaneously modified;
- at most one of positions 976, 982 and 983 is modified;
- positions 1048 and 1056 are not simultaneously modified
- positions 1056 and 1060 are not simultaneously modified; and
- positions 1212 and 1214 are not simultaneously modified.

Another object of the present invention is a modified protein derived from a nucleocapside protein (N) of SARS-CoV-2, characterized in that it comprises at least 2, preferably at least 3, more preferably at least 4 modifications selected amongst Q9Y, A35Y, L45A, P80T, R88Y, D103K, S105Y, T148Y, N150Y, N150A, S190Y, S202Y, Q229K, L291K, Q303Y, G316Y, W330K, K374Y and K387Y, with the proviso that:
- positions 103 and 105 are not simultaneously modified; and
- positions 148 and 150 are not simultaneously modified.

Immunogenic composition comprising such a modified S protein and/or a modified N protein, or nucleic acid(s) encoding the same, is also part of the present invention.

### DETAILED DESCRIPTION OF THE PREFERRED EMBODIMENTS

T cell immune response against a viral antigen is restricted to some epitopes named immunodominants. The main property of the CD8 T cell immunodominant epitopes is their high affinity for the restricting HLA-I molecule.

Variant strains that have mutated immunodominant epitopes are no more recognized by the T cells elicited by the initial strain and the current vaccines and may be immune escape variants.

Other epitopes with lower affinity for HLA-I, called "subdominant" or "cryptic", cannot induce a CD8 response but they can be recognized by already activated CD8 T cells.

Since cryptic epitopes are not subject to selection pressure, the initial strain and the immune escape variants should share the same cryptic epitopes and should all be sensitive to CD8 T cells specific for these epitopes.

The present invention thus aims at providing a new vaccine, which is able to induce an immune response against several cryptic epitopes of the S protein and/or the N protein of SARS-CoV-2.

In order to be efficient for a significant part of the population, this vaccine must induce an immune response against cryptic epitopes presented by the major HLA-I molecules HLA-A1, -A2, -A3, -A24, -B7, -B35 and Cw7, that cover more than 90% of people (Table 3).

**Table 3: Frequency of human population expressing at least one of the HLA-A1, -A2, - A3, -A24, -B7, -B35 and CW7 molecules (percentages calculated from the data available in the Allele Frequency Net Database (AFND)**

| EU | North Africa | Sub-saharian Africa | North America | North-Est Asia | South-Central America | South Asia | South-Est Asia | West Asia |
|---|---|---|---|---|---|---|---|---|
| 96% | 91% | 94% | 96% | 97% | 90% | 95% | 95% | 92% |

This vaccine should maintain the capacity to generate neutralizing antibodies against the RBD of the protein S.

According to a first aspect, the present invention pertains to a modified protein derived from a spike (S) protein of SARS-CoV-2 and comprises a number of modifications, wherein each of these modifications is likely to increase the affinity of a cryptic epitope presented by one of the major HLA-I molecules (HLA-A1, -A2, -A3, -A24, -B7, -B35 and Cw7) for this HLA-I molecule. An epitope resulting from such a modification is herein called an "optimized epitope".

More precisely, the modified S protein comprises at least 4, preferably at least 5, more preferably at least 6 modifications selected from the group consisting of: L24A, K77Y, T109Y, D138Y, M153T, E169R, Q173Y, 1197T, I714Y, I714A, S721Y, E725K, L727Y, S810Y, S813Y, K814Y, L821Y, G838T, Q853Y, G857Y, V915Y, S937Y, V976Y, S982Y, R983Y, V1040T, H1048Y, A1056Y, V1060Y, A1087Y, I1183T, N1192Y, W1212A, M1214R, G1223Y and V1264K, with the proviso that:
- positions 169 and 173 are not simultaneously modified;
- positions 714 and 721 are not simultaneously modified;
- at most one of positions 721, 725 and 727 is modified;
- at most one of positions 810, 813 and 814 is modified;
- at most one of positions 813, 814 and 821 is modified;
- positions 853 and 857 are not simultaneously modified;
- at most one of positions 976, 982 and 983 is modified;
- positions 1048 and 1056 are not simultaneously modified
- positions 1056 and 1060 are not simultaneously modified; and
- positions 1212 and 1214 are not simultaneously modified.

In what precedes, the modifications are indicated having regard to the sequence of the S protein of the initial strain (SEQ ID No: 1), but the modified S protein of the invention can obviously be derived from the S protein of any existing or future variant of SARS-CoV-2.

The reason why certain modifications cannot be present in the same modified S protein is that modifications in overlapping epitopes should be avoided to maintain the capacity of each optimized epitope to elicit a CTL response that cross-recognizes its cognate native epitope.

A generic sequence of some possible variants of protein S according to the invention is shown in SEQ ID No: 2. This sequence comprises two types of variable residues: (i) residues which can be modified to increase the affinity of a cryptic epitope with a HLA molecule (at positions 24, 77, 109, 138, 153, 169, 173, 197, 215, 246, 714, 721, 725, 727, 810, 813, 814, 821, 838, 853, 857, 915, 937, 976, 982, 983, 1040, 1048, 1056, 1060, 1087, 1183, 1192, 1212, 1214, 1223 and 1264) and (ii) residues corresponding to mutations which have been found in at least one variant to date; such mutations will preferably not be introduced in an optimized epitope of the modified protein according to the invention. Of course, the skilled person can introduce one or more additional mutations in the modified S protein of the invention, especially in the RBD, to adapt the sequence to any new variant.

More generally, the modified S protein according to the present invention comprises a sequence having at least 90%, preferably at least 95% and more preferably 98 to 100% identity with residues 1 to 199 of SEQ ID No: 2, and/or a sequence having at least 90%, preferably at least 95% and more preferably 98 to 100% identity with residues 700 to 1273 of SEQ ID No: 2. In a modified S protein according to the invention, the part of the S1 subunit from amino acid 200 to 699 (which encompasses the RBD region) can be easily adapted by the skilled person to elicit a humoral immune response against the RBD of any newly emerging variant (typically, by introducing mutations observed in such a variant).

The skilled person can, by routine experimentation as described in Examples 4-6, confirm the immunogenicity of the different epitopes of SEQ ID Nos: 5-118 and test different versions of the S protein, combining modifications to optimize epitopes presented by different HLA molecules, in order to obtain sequences that elicit cellular responses in most patients, targeting as many cryptic epitopes of the S protein as possible. As already mentioned, the skilled person will select the positions to modify such that two modifications do not occur in the same epitope. Modifications provoking an important change in the tridimensional structure of the protein will also be avoided.

When selecting the modifications, the skilled person will preferably choose those which transform a cryptic epitope that weakly binds to a HLA molecule into an epitope that very strongly binds to the same HLA molecule. Hence, according to a particular embodiment, the modified S protein comprises at least 2 modifications selected from the group consisting of: D138Y, E169R, Q173Y, I197T, E725K, G838T, Q853Y and N1192Y. For example, the modified S protein comprises 3, 4, 5, 6 or 7 of these modifications.

According to another particular embodiment, the modified S protein of the invention comprises at least 1 modification selected from the group consisting of: K77Y, M153T, L821Y, V976Y, R983Y, H1048Y and V1060Y. For example, it can comprise 1, 2, 3, 4, 5 or 6 of these modifications, possibily combined to one or several modifications selected amongst D138Y, E169R, Q173Y, I197T, E725K, G838T, Q853Y and N1192Y.

According to another particular embodiment, the modified S protein of the invention comprises at least 1 modification selected from the group consisting of: T109Y, I714Y, S721Y, S810Y, S813Y, K814Y, G857Y, V915Y, V1040T, A1056Y, A1087Y and I1183T. For example, it can comprise 1, 2, 3, 4, 5, 6, 7, 8 or 9 of these modifications, possibily combined to one or several modifications selected amongst D138Y, E169R, Q173Y, I197T, E725K, G838T, Q853Y and N1192Y, and/or to one or several modifications selected amongst K77Y, M153T, L821Y, V976Y, R983Y, H1048Y and V1060Y.

According to another particular embodiment of the invention, the modified S protein is as any of the above-described embodiments and further comprises the modification I714A and/or the modification L727Y.

According to another particular embodiment of the invention, the modified S protein is as any of the above-described embodiments and further comprises at least 1 modification for example 1, 2, 3 or 4 modifications selected amongst L24A, S937Y, S982Y, W1212A, M1214R, G1223Y and V1264K.

In order to elicit a CTL response in a large section of the population, the modified S protein according to the invention preferably comprises optimized epitopes presented by several of the major HLA-I molecules HLA-A1, -A2, -A3, -A24, -B7, -B35 and Cw7. Hence, according to a particular embodiment, the modified S protein comprises modifications selected from at least 2, preferably at least 3, more preferably at least 4 and even more preferably 5, 6 or 7 of the following groups:
(i) group A2: T109Y, S721Y, L821Y, G857Y, V915Y, S937Y, V976Y, R983Y, H1048Y, V1060Y, N1192Y and G1223Y;
(ii) group B7: L24A, I714A and W1212A;
(iii) group A1: M153T, I197T, G838T, V1040T and I1183T;
(iv) group A3: E725K and V1264K;
(v) group A24: E169R and M1214R;
(vi) group B35: D138Y, Q173Y, I714Y, L727Yand A1056Y in S protein; and
(vii) group Cw7: K77Y, S810Y, S813Y, K814Y, Q853Y, S982Y and A1087Y.

The present invention also pertains to a modified protein derived from a nucleocapside protein (N) of SARS-CoV-2, characterized in that it comprises at least 2, preferably at least 3, more preferably at least 4 modifications selected from the group consisting of Q9Y, A35Y, L45A, P80T, R88Y, D103K, S105Y, T148Y, N150Y, N150A, S190Y, S202Y, Q229K, L291K, Q303Y, G316Y, W330K, K374Y and K387Y, with the proviso that:
- positions 103 and 105 are not simultaneously modified; and
- positions 148 and 150 are not simultaneously modified.

In the above, the modifications in N protein are indicated having regard to the sequence of the N protein of the initial strain (SEQ ID No: 3), but the modified N protein of the invention can obviously be derived from the N protein of any existing or future variant of SARS-CoV-2.

As in the S protein, modifications in overlapping epitopes in the N protein should be avoided to maintain the capacity of each optimized epitope to elicit a CTL response that cross-recognizes its cognate native epitope.

A generic sequence of some possible modified N proteins according to the invention is shown in SEQ ID No: 4, which comprises (i) variable residues corresponding to modifications which can be introduced to increase the affinity of a cryptic epitope with a HLA molecule (at positions 9, 35, 45, 80, 88, 103, 105, 148, 150, 190, 202, 229, 291, 303, 316, 330, 374 and 387) and (ii) residues corresponding to mutations which have been found in at least one variant to date. Of course, the skilled person can introduce one or more additional mutations in the modified N protein of the invention, to adapt the sequence to any new variant, but preferably not in the optimized epitopes.

More generally, the modified N protein according to the present invention comprises a sequence having at least 90%, preferably at least 95% and more preferably 98 to 100% identity with SEQ ID No: 4.

The skilled person can, by routine experimentation as described in Examples 4-6, confirm the immunogenicity of the different epitopes of SEQ ID Nos: 119-156 and test different versions of the modified N protein, combining modifications to optimize epitopes presented by different HLA molecules, in order to obtain sequences that elicit cellular responses in most patients, targeting as many cryptic epitopes of the N protein as possible. As already mentioned, the skilled person will select the positions to modify such that two modifications do not occur in the same epitope. Modifications provoking an important change in the tridimensional structure of the protein will also be avoided.

When selecting the modifications, the skilled person will preferably choose those which transform a cryptic epitope that weakly binds to a HLA molecule into an epitope that very strongly binds to the same HLA molecule. Hence, according to a particular embodiment, the modified N protein comprises at least 1, preferably 2 modifications selected amongst Q9Y, P80T, G316Y and K387Y. The modified N protein can comprise 1, 2, 3 or 4 of these modifications.

According to another particular embodiment, the modified N protein of the invention comprises at least 1, preferably at least 2 modifications selected from the group consisting of: A35Y, D103K, T148Y, N150A, S190Y, S202Y, Q229K and K374Y. For example, it can comprise 1, 2, 3, 4, 5 or 6 of these modifications, possibily combined to one or several modifications selected amongst Q9Y, P80T, G316Y and K387Y.

According to another particular embodiment of the invention, the modified N protein is as any modified N protein described above and comprises at least 1 modification, for example 1, 2, 3, 4, 5, 6 or 7 modifications selected amongst L45A, R88Y, S105Y, N150Y, L291K, Q303Y and W330K.

When performing the invention, the skilled person can choose any convenient way to administer the modified S protein and/or the modified N protein.

This modified S protein and/or the modified N protein could be administered as encapsulated mRNA, vectorized cDNA or recombinant proteins along with an adjuvant as is the case of other vaccines against SARS-CoV-2 (Poland et al., 2020).

The present invention thus also pertains to a nucleic acid molecule encoding a modified S protein and/or a modified N protein as above-described.

According to a particular embodiment, the nucleic acid molecule is a DNA molecule. Such a DNA molecule can comprise cDNA sequence(s) encoding the modified S and/or N protein(s), under the control of appropriate promoter(s). The skilled person can choose any appropriate vector for administering this DNA molecule.

According to another particular embodiment, the nucleic acid molecule is an mRNa molecule. The skilled person can for example adapt the mRNA vaccine technology recently used in mRNA-2173 (Moderna) or in BNT162b2 (Pfizer/BioNTech) to obtain a vaccine leading to the expression of a modified S protein and/or a modified N protein according to the present invention.

According to another particular embodiment, the S and/or N modified proteins are administered as purified proteins, in a formulation appropriate for vaccination in humans. By "formulation appropriate for vaccination in humans" is herein meant that the proteins are in a medium/buffer/solution which is pharmaceutically acceptable. Such a formulation can also comprise appropriate adjuvant (e.g., a mixture of a highly purified mineral oil such as Drakeol 6VR and a surfactant such as mannide monooleate - such as Montanide) and/or immunstimulant(s) (e.g., Poly(I:C) and CpG-ODN).

The present invention also pertains to an immunogenic composition comprising a modified S protein and/or a modified N protein as above-described, or a nucleic acid or nucleic acids encoding the same.

By "immunogenic composition" is herein meant a composition which elicits an immune response against the S and/or N proteins when administered to human individuals. In a preferred embodiment, the immunogenic composition elicits both a humoral response and a CTL response against the S and/or N proteins in most human individuals.

The immunogenic composition can be formulated for any appropriate route of administration (e.g., transcutaneous, intramuscular, nasal and/or lung spray, etc.)

According to a particular embodiment, the immunogenic composition comprises both a modified S protein and a modified N protein as above-described.

In such a case, in order to efficiently elicit CTL responses in a majority of individuals, the immunogenic composition preferably includes modified S and N proteins comprising modifications selected from at least 2, preferably at least 3, more preferably at least 4 and even more preferably 5, 6 or 7 of the following groups:
(i) group A2: T109Y, S721Y, L821Y, G857Y, V915Y, S937Y, V976Y, R983Y, H1048Y, V1060Y, N1192Y and G1223Y in S protein and Q303Y and G316Y in N protein;
(ii) group B7: L24A, I714A and W1212A in S protein and L45A and N150A in N protein;
(iii) group A1: M153T, I197T, G838T, V1040T and I1183T in S protein and P80T in N protein;
(iv) the group A3: E725Kand V1264K in S protein and Q229K, L291K, D103K and W330K in N protein;
(v) group A24: E169R and M1214R in S protein;
(vi) group B35: D138Y, Q173Y, I714Y, L727Yand A1056Y in S protein and S105Y and N150Y in N protein; and
(vii) group Cw7: K77Y, S810Y, S813Y, K814Y, Q853Y, S982Y and A1087Y in S protein and Q9Y, A35Y, R88Y, T148Y, S190Y, S202Y, K374Y and K387Y in N protein.

Other characteristics of the invention will also become apparent in the course of the description which follows of the assays which have been and are performed in the framework of the invention and which provide it with the required experimental support, without limiting its scope.

### EXAMPLES

### Example 1: Identification of cryptic epitopes in S and N proteins and design of optimized epitopes able to elicit a CTL response against these

Cryptic CD8 epitopes are unable to trigger an immune response because of their low affinity for the presenting HLA-I molecule. The best way to increase their immunogenicity is to improve their affinity for HLA-I. This can be achieved by replacing amino acids which are unfavorable for binding to HLA-I by amino acids that are favorable. However, these modifications should maintain unchanged the segment of the epitope that interacts with the T cell receptor (TCR). This is a prerequisite for the induced CD8 cells to recognize the wild type epitope expressed by the virus. Previous works have shown that the best position of the epitope to modify is the N-terminal position.

Using the NetMHC algorithm, we identified epitopes derived from protein S and N that should have a low affinity for the different HLA-I of interest. We modified them at the N-terminal or second position to increase their binding affinity (Tables 4 and 5).

Epitopes are classified as VSB (very strong binders) (% rank<0.10), SB (strong binders) (% rank 0.10-0.40), WB/SB (weak binders/strong binders) (% rank 0.40-0.50) and WB (weak binders) (% rank >0.50).

**Table 4: native and putative optimized epitopes in protein S**

| 1^{st} position of the epitope | HLA allele | Sequence | Seq ID No: | % Rank | Binding strength |
|---|---|---|---|---|---|
| 109 | HLA-A0201 | TLDSKTQSL | 5 | 1.40 | WB |
| | | YLDSKTQSL | 6 | 0.12 | SB |
| 202 | HLA-A0201 | KIYSKHTPI | 7 | 2.00 | WB |
| | | YIYSKHTPI | 8 | 0.50 | WB/SB |
| 417 | HLA-A0201 | KIADYNYKL | 9 | 0.50 | WB/SB |
| | | YIADYNYKL | 10 | 0.10 | VSB |
| 721 | HLA-A0201 | SVTTEILPV | 11 | 1.10 | WB |
| | | YVTTEILPV | 12 | 0.15 | SB |
| 821 | HLA-A0201 | LLFNKVTLA | 13 | 0.40 | WB/SB |
| | | YLFNKVTLA | 14 | 0.05 | VSB |
| 857 | HLA-A0201 | GLTVLPPLL | 15 | 1.30 | WB |
| | | YLTVLPPLL | 16 | 0.15 | SB |
| 915 | HLA-A0201 | VLYENQKLI | 17 | 1.60 | WB |
| | | YLYENQKLI | 18 | 0.30 | SB |
| 937 | HLA-A0201 | SLSSTASAL | 19 | 2.00 | WB |
| | | YLSSTASAL | 20 | 0.40 | WB/SB |
| 976 | HLA-A0201 | VLNDILSRL | 21 | 0.50 | WB/SB |
| | | YLNDILSRL | 22 | 0.04 | VSB |
| 983 | HLA-A0201 | RLDKVEAEV | 23 | 0.50 | WB/SB |
| | | YLDKVEAEV | 24 | 0.06 | VSB |
| 1048 | HLA-A0201 | HLMSFPQSA | 25 | 0.40 | WB/SB |
| | | YLMSFPQSA | 26 | 0.01 | VSB |
| 1060 | HLA-A0201 | VVFLHVTYV | 27 | 0.50 | WB/SB |
| | | YVFLHVTYV | 28 | 0.05 | VSB |
| 1192 | HLA-A0201 | NLNESLIDL | 29 | 1.50 | WB |
| | | YLNESLIDL | 30 | 0.09 | VSB |
| 1223 | HLA-A0201 | GLIAIVMVT | 31 | 1.80 | WB |
| | | YLIAIVMVT | 32 | 0.40 | WB/SB |
| 24 | HLA-B0702 | LPPAYTNSF | 33 | 0.60 | WB |
| | | APPAYTNSF | 34 | 0.40 | WB/SB |
| 208 | HLA-B0702 | TPINLVRDL | 35 | 0.70 | WB |
| | | APINLVRDL | 36 | 0.30 | SB |
| 526 | HLA-B0702 | GPKKSTNLV | 37 | 1.40 | WB |
| | | APKKSTNLV | 38 | 0.50 | WB/SB |
| 714 | HLA-B0702 | IPTNFTISV | 39 | 0.40 | WB/SB |
| | | APTNFTISV | 40 | 0.25 | SB |
| 1212 | HLA-B0702 | WPWYIWLGF | 41 | 0.70 | WB |
| | | APWYIWLGF | 42 | 0.50 | WB/SB |
| 169 | H LA-A2402 | EYVSQPFLM | 43 | 0.60 | WB |
| | | RYVSQPFLM | 44 | 0.06 | VSB |
| 264 | H LA-A2402 | AYYVGYLQP | 45 | 1.90 | WB |
| | | RYYVGYLQP | 46 | 0.50 | WB/SB |
| 1214 | H LA-A2402 | WYIWLGFIA | 47 | 1.50 | WB |
| | | RYIWLGFIA | 48 | 0.40 | WB/SB |
| 138 | HLA-B3501 | DPFLGVYYH | 49 | 0.80 | WB |
| | | YPFLGVYYH | 50 | 0.09 | VSB |
| 173 | HLA-B3501 | QPFLMDLEG | 51 | 0.70 | WB |
| | | YPFLMDLEG | 52 | 0.10 | VSB |
| 229 | HLA-B3501 | LPIGINITR | 53 | 0.70 | WB |
| | | YPIGINITR | 54 | 0.40 | WB/SB |
| 271 | HLA-B3501 | QPRTFLLKY | 55 | 0.60 | WB |
| | | YPRTFLLKY | 56 | 0.08 | VSB |
| 321 | HLA-B3501 | QPTESIVRF | 57 | 0.40 | WB/SB |
| | | YPTESIVRF | 58 | 0.04 | VSB |
| 336 | HLA-B3501 | CPFGEVFNA | 59 | 2.00 | WB |
| | | YPFGEVFNA | 60 | 0.50 | WB/SB |
| 478 | HLA-B3501 | TPCNGVEGF | 61 | 1.00 | WB |
| | | YPCNGVEGF | 62 | 0.17 | SB |
| 664 | HLA-B3501 | IPIGAGICA | 63 | 0.90 | WB |
| | | YPIGAGICA | 64 | 0.25 | SB |
| 714 | HLA-B3501 | IPTNFTISV | 65 | 0.70 | WB |
| | | YPTNFTISV | 66 | 0.17 | SB |
| 727 | HLA-B3501 | LPVSMTKTS | 67 | 0.40 | WB/SB |
| | | YPVSMTKTS | 68 | 0.17 | SB |
| 1056 | HLA-B3501 | APHGVVFLH | 69 | 1.80 | WB |
| | | YPHGVVFLH | 70 | 0.17 | SB |
| 77 | HLA-C0701 | KRFDNPVLP | 71 | 0.40 | WB/SB |
| | | YRFDNPVLP | 72 | 0.03 | VSB |
| 205 | HLA-C0701 | SKHTPINLV | 73 | 1.00 | WB |
| | | YKHTPINLV | 74 | 0.12 | SB |
| 354 | HLA-C0701 | NRKRISNCV | 75 | 1.30 | WB |
| | | YRKRISNCV | 76 | 0.08 | SB |
| 810 | HLA-C0701 | SKPSKRSFI | 77 | 0.70 | WB |
| | | YKPSKRSFI | 78 | 0.15 | SB |
| 813 | HLA-C0701 | SKRSFIELD | 79 | 1.50 | WB |
| | | YKRSFIELD | 80 | 0.17 | SB |
| 814 | HLA-C0701 | KRSFIEDLL | 81 | 1.20 | WB |
| | | YRSFIEDLL | 82 | 0.12 | SB |
| 853 | HLA-C0701 | QKFNGLTVL | 83 | 0.80 | WB |
| | | YKFNGLTVL | 84 | 0.07 | VSB |
| 982 | HLA-C0701 | SRLDKVEAE | 85 | 1.70 | WB |
| | | YRLDKVEAE | 86 | 0.40 | WB/SB |
| 1087 | HLA-C0701 | AHFPREGVF | 87 | 1.50 | WB |
| | | YHFPREGVF | 88 | 0.30 | SB |
| 152 | HLA-A0101 | WMESEFRVY | 89 | 0.40 | WB/SB |
| | | WTESEFRVY | 90 | 0.07 | VSB |
| 196 | HLA-A0101 | NIDGYFKIY | 91 | 0.90 | WB |
| | | NTDGYFKIY | 92 | 0.07 | VSB |
| 465 | HLA-A0101 | ERDISTEIY | 93 | 0.80 | WB |
| | | ETDISTEIY | 94 | 0.01 | VSB |
| 482 | HLA-A0101 | GVEGFNCYF | 95 | 1.90 | WB |
| | | GTEGFNCYF | 96 | 0.40 | WB/SB |
| 584 | HLA-A0101 | ILDITPCSF | 97 | 1.00 | WB |
| | | ITDITPCSF | 98 | 0.17 | SB |
| 612 | HLA-A0101 | YQDVNCTEV | 99 | 2.00 | WB |
| | | YTDVNCTEV | 100 | 0.05 | VSB |
| 652 | HLA-A0101 | GAEHVNNSY | 101 | 0.90 | WB |
| | | GTEHVNNSY | 102 | 0.06 | VSB |
| 837 | HLA-A0101 | YGDCLGDIA | 103 | 1.80 | WB |
| | | YTDCLGDIA | 104 | 0.04 | VSB |
| 1039 | HLA-A0101 | RVDFCGKGY | 105 | 1.50 | WB |
| | | RTDFCGKGY | 106 | 0.25 | SB |
| 1182 | HLA-A0101 | EIDRLNEVA | 107 | 1.50 | WB |
| | | ETDRLNEVA | 108 | 0.15 | SB |
| 311 | HLA-A0301 | GIYQTSNFR | 109 | 0.60 | WB |
| | | KIYQTSNFR | 110 | 0.20 | SB |
| 349 | HLA-A0301 | SVYAWNRKR | 111 | 1.00 | WB |
| | | KVYAWNRKR | 112 | 0.40 | WB/SB |
| 409 | HLA-A0301 | QIAPGQTGK | 113 | 0.60 | WB |
| | | KIAPGQTGK | 114 | 0.12 | SB |
| 725 | HLA-A0301 | EILPVSMTK | 115 | 1.00 | WB |
| | | KILPVSMTK | 116 | 0.02 | VSB |
| 1264 | HLA-A0301 | VLKGVKLHY | 117 | 1.30 | WB |
| | | KLKGVKLHY | 118 | 0.50 | WB/SB |

**Table 5 : native and putative optimized epitopes in protein N**

| 1^{st} position of the epitope | HLA allele | Sequence | Seq ID No: | % Rank | Binding strength |
|---|---|---|---|---|---|
| 303 | HLA-A0201 | QIAQFAPSA | 119 | 6.00 | WB |
| | | YIAQFAPSA | 120 | 0.50 | WB/SB |
| 316 | HLA-A0201 | GMSRIGMEV | 121 | 0.60 | WB |
| | | YMSRIGMEV | 122 | 0.06 | VSB |
| 45 | HLA-B0702 | LPNNTASWF | 123 | 0.80 | WB |
| | | APNNTASWF | 124 | 0.50 | WB/SB |
| 150 | HLA-B0702 | NPANNAAIV | 125 | 0.70 | WB |
| | | APANNAAIV | 126 | 0.25 | SB |
| 79 | HLA-A0101 | SPDDQIGYY | 127 | 1.80 | WB |
| | | STDDQIGYY | 128 | 0.01 | VSB |
| 229 | HLA-A0301 | QLESKMSGK | 129 | 1.00 | WB |
| | | KLESKMSGK | 130 | 0.20 | SB |
| 291 | HLA-A0301 | LIRQGTDYK | 131 | 0.90 | WB |
| | | KIRQGTDYK | 132 | 0.40 | WB/SB |
| 103 | HLA-A0301 | DLSPRWYFY | 133 | 5.50 | WB |
| | | KLSPRWYFY | 134 | 0.15 | SB |
| 330 | HLA-A0301 | WLTYTGAIK | 135 | 2.50 | WB |
| | | KLTYTGAIK | 136 | 0.40 | WB/SB |
| 105 | HLA-B3501 | SPRWYFYYL | 137 | 2.00 | WB |
| | | YPRWYFYYL | 138 | 0.40 | WB/SB |
| 150 | HLA-B3501 | NPANNAAIV | 139 | 2.50 | WB |
| | | YPANNAAIV | 140 | 0.40 | WB/SB |
| 9 | HLA-C0701 | QRNAPRITF | 141 | 0.60 | WB |
| | | YRNAPRITF | 142 | 0.03 | VSB |
| 35 | HLA-C0701 | ARSKQRRPQ | 143 | 1.30 | WB |
| | | YRSKQRRPQ | 144 | 0.17 | SB |
| 88 | HLA-C0701 | RRATRRIRG | 145 | 2.00 | WB |
| | | YRATRRIRG | 146 | 0.50 | WB/SB |
| 148 | HLA-C0701 | TRNPANNAA | 147 | 1.80 | WB |
| | | YRNPANNAA | 148 | 0.30 | SB |
| 190 | HLA-C0701 | SRNSSRNST | 149 | 1.00 | WB |
| | | YRNSSRNST | 150 | 0.12 | SB |
| 202 | HLA-C0701 | SRGTSPARM | 151 | 1.20 | WB |
| | | YRGTSPARM | 152 | 0.15 | SB |
| 374 | HLA-C0701 | KKADETQAL | 153 | 2.00 | WB |
| | | YKADETQAL | 154 | 0.20 | SB |
| 387 | HLA-C0701 | KKQQTVTLL | 155 | 1.30 | WB |
| | | YKQQTVTLL | 156 | 0.09 | VSB |

### Example 2: classifying the proposed modifications in protein S

From the data shown in Table 3, we classified the proposed modifications in 5 different groups. The first and 3^{rd} groups comprise the modifications most likely to create epitopes able to elicit a CTL response that will recognize their native cognate cryptic epitope. The first group is likely to induce a stronger immune response than the 3^{rd} group. In the second and 4^{th} groups, the wild-type epitopes might be immunodominant (i.e., not cryptic). In the 5^{th} group, the optimized epitope might not be immunogenic.

This classification is shown in the following tables 6 to 11.

The aa 200-685 segment of the protein S, that contains the RBD target of neutralizing Ab, will be kept unchanged.

We will modify the 1-200 aa of the S1 segment and the whole S2 segment of the protein S in order to create optimized cryptic epitopes able to trigger a CD8 immune response.

This new vaccine should therefore induce a humoral response to the RBD of the initial strain and any other variant that has not mutated this region in such a way that it escapes the humoral immunity triggered by the initial strain, and a polyspecific/poly HLA-I CD8 response against the initial strain and all other variants even those having mutated the RDB region and that are no more recognized by neutralizing antibodies.

Alternatively, the segment of aa 200-685 of the subdomain S1 can be designed to comprise mutations observed in emerging variants, especially when these mutations enable the virus to escape the humoral response developed against the initial strain. It can even comprise mutations which have not been observed simultaneously in a single strain, to create a chimeric RBD which will trigger a humoral response against several strains.

**Table 6: Group 1 - modifications creating VSB epitopes derived from WB wild type epitopes**

| HLA | Peptide |
|---|---|
| A2 | 1192 |
| B7 | |
| A24 | 169 |
| B35 | 138, 173 |
| Cw7 | 853 |
| A1 | 196, 837 |
| A3 | 725 |

**Table 7: Goup 2 - modifications creating VSB epitopes derived from SB/WB epitopes**

| HLA | Peptide |
|---|---|
| A2 | 821, 976, 983, 1048, 1060 |
| B7 | |
| A24 | |
| B35 | |
| Cw7 | 77 |
| A1 | 152 |
| A3 | |

**Table 8: Group 3 - modifications creating SB derived from WB wild type epitopes**

| HLA | Peptide |
|---|---|
| A2 | 109, 721, 857, 915 |
| B7 | |
| A24 | |
| B35 | 714, 1056 |
| Cw7 | 810, 813, 814, 1087 |
| A1 | 1040, 1183 |
| A3 | |

**Table 9: Group 4 - modifications creating SB derived from WB/SB wild type epitopes**

| HLA | Peptide |
|---|---|
| A2 | |
| B7 | 714 |
| A24 | |
| B35 | 727 |
| Cw7 | |
| A1 | |
| A3 | |

**Table 10: Group 5 - modifications creating WB/SB epitopes derived from WB wild type epitopes**

| HLA | Peptide |
|---|---|
| A2 | 937, 1223 |
| B7 | 24, 1212 |
| A24 | 1214 |
| B35 | |
| Cw7 | 982 |
| A1 | |
| A3 | 1264 |

The modifications will be chosen to elicit CTLs against as many cryptic epitopes as possible (it being understood that the skilled person will avoid introducing modifications likely to provoke self-immunity or any other side effect), specific for as many HLA molecules as possible and without any deleterious impact on the tridimesional structure of the S protein. Of course, two modifications will not be introduced in overlapping epitopes in the same molecule. The following table summarizes the modifications proposed in the S protein, as well as the mutual exclusions between these modifications.

**Table 11: proposed modifications in the S protein. The two last columns show the modifications which cannot be introduced in a same molecule. *: modification in the second position of the HLA-A1 epitope. 1: VSB from WB; 2: VSB from SB/WB; 3: SB from WB; 4: SB from WB/SB; 5: WB/SB from WB**

| HLA | position | modification | 1 | 2 | 3 | 4 | 5 | Exclusions | |
|---|---|---|---|---|---|---|---|---|---|
| B0702 | 24 | L->A | | | | | X | | |
| C0701 | 77 | K->Y | | X | | | | | |
| A0201 | 109 | T->Y | | | X | | | | |
| B3501 | 138 | D->Y | X | | | | | | |
| A0101 | 153* | M->T | | X | | | | | |
| A2402 | 169 | E->R | X | | | | | mutually exclusive | |
| B3501 | 173 | Q->Y | X | | | | | | |
| A0101 | 197* | I->T | X | | | | | | |
| B3501 | 714 | I->Y | | | X | | | mutually exclusive | |
| B0702 | 714 | I->A | | | | X | | | |
| A0201 | 721 | S->Y | | | X | | | | mutually exclusive |
| A0301 | 725 | E->K | X | | | | | | |
| B3501 | 727 | L->Y | | | | X | | | |
| C0701 | 810 | S->Y | | | X | | | mutually exclusive | |
| C0701 | 813 | S->Y | | | X | | | | mutually exclusive |
| C0701 | 814 | K->Y | | | X | | | | |
| A0201 | 821 | L->Y | | X | | | | | |
| A0101 | 838* | G->T | X | | | | | | |
| C0701 | 853 | Q->Y | X | | | | | mutually exclusive | |
| A0201 | 857 | G->Y | | | X | | | | |
| A0201 | 915 | V->Y | | | X | | | | |
| A0201 | 937 | S->Y | | | | | X | | |
| A0201 | 976 | V->Y | | X | | | | | |
| C0701 | 982 | S->Y | | | | | X | mutually exclusive | |
| A0201 | 983 | R->Y | | X | | | | | |
| A0101 | 1040* | V->T | | | X | | | | |
| A0201 | 1048 | H->Y | | X | | | | mutually exclusive | |
| B3501 | 1056 | A->Y | | | X | | | | mutually exclusive |
| A0201 | 1060 | V->Y | | X | | | | | |
| C0701 | 1087 | A->Y | | | X | | | | |
| A0101 | 1183* | I->T | | | X | | | | |
| A0201 | 1192 | N->Y | X | | | | | | |
| B0702 | 1212 | W->A | | | | | X | mutually exclusive | |
| A2402 | 1214 | M->R | | | | | X | | |
| A0201 | 1223 | G->Y | | | | | X | | |
| A0301 | 1264 | V->K | | | | | X | | |

### Example 3: classifying the proposed modifications in protein N

From the data shown in Table 4, we classified the proposed modifications in 3 different groups. The first and 2^{nd} groups comprise the modifications most likely to create epitopes able to elicit a CTL response that will recognize their native cognate cryptic epitope. The first group is likely to induce a stronger immune response than the 2^{nd} group. The 3^{rd} one comprises modifications creating optimized epitopes that might not be immunogenic.

This classification is shown in the following tables 12 to 15. Modifications can be introduced in any region of the N protein.

**Table 12: Group 1 - modifications leading to VSB epitopes derived from WB wild type epitopes**

| HLA | Peptide |
|---|---|
| A2 | 316 |
| B7 | |
| A24 | |
| B35 | |
| Cw7 | 9, 387 |
| A1 | 79 |
| A3 | |

**Table 13: Group 2 - modifications creating SB epitopes derived from WB wild type epitopes**

| HLA | Peptide |
|---|---|
| A2 | |
| B7 | 150 |
| A24 | |
| B35 | |
| Cw7 | 35, 148, 190, 202, 374, |
| A1 | |
| A3 | 103, 229 |

**Table 14: Group 3 - modifications creating WB/SB epitopes derived from WB wild type epitopes**

| HLA | Peptide |
|---|---|
| A2 | 303 |
| B7 | 45 |
| A24 | |
| B35 | 105, 150 |
| Cw7 | 88 |
| A1 | |
| A3 | 291, 330 |

The following table summarizes the modifications proposed in the N protein, as well as the mutual exclusions between these modifications.

**Table 15: proposed modifications in the N protein. The last column shows the modifications which cannot be introduced in a same molecule. *: modification in the second position of the HLA-A1 epitope. 1: VSB from WB; 2: SB from WB; 3: WB/SB from WB.**

| HLA | position | modification | 1 | 2 | 3 | Exclusions |
|---|---|---|---|---|---|---|
| C0701 | 9 | Q→Y | X | | | |
| C0701 | 35 | A→Y | | X | | |
| B0702 | 45 | L→A | | | X | |
| A0101 | 80* | P→T | X | | | |
| C0701 | 88 | R→Y | | | X | |
| A0301 | 103 | D→K | | X | | mutually exclusive |
| B3501 | 105 | S→Y | | | X | |
| C0701 | 148 | T→Y | | X | | mutually exclusive |
| B3501 | 150 | N→Y | | | X | |
| B0702 | 150 | N→A | | X | | |
| C0701 | 190 | S→Y | | X | | |
| C0701 | 202 | S→Y | | X | | |
| A0301 | 229 | Q→K | | X | | |
| A0301 | 291 | L→K | | | X | |
| A0201 | 303 | Q→Y | | | X | |
| A0201 | 316 | G→Y | X | | | |
| A0301 | 330 | W→K | | | X | |
| C0701 | 374 | K→Y | | X | | |
| C0701 | 387 | K→Y | X | | | |

### Example 4: Confirmation of the predicted HLA binding affinity of all selected WT and optimized peptides

We use the very common technic of the HLA-peptide binding assay for peptides predicted to bind to HLA-A*0101, HLA-A*0201, HLA-A*0301, HLA-A*2402, HLA-B*0702, HLA-B*3501 and HLA-C*0701.
It consists in comparing the binding affinity of a tested peptide to the affinity of a known strong binder peptide for the same HLA molecule. This technic is well described (Tourdot et al., 2000; Scardino et al., 2004; Gallou et al., 2006).

### Example 5: Identification of immunodominant epitopes

Since all the immunodominant epitopes are binders to HLA but all the binders may not be immunodominant we identify immunodominant epitopes by evaluating the reactivity of T cells from SARS-CoV-2 infected patients against at least the WT peptides that the HLA-peptide binding test showed to be strong or very strong binders. We use the very common IFNg ELISpot assay.

### Example 6: Recognition of the different versions of optimized proteins S by antibodies present in the serum of infected patients

To confirm that the modifications we introduce in the sequences of the protein S do not alter the recognition of this modified protein S by the neutralizing antibodies generated in patients infected by the initial strain, we produce the recombinant WT protein S and each of its selected modified versions and test the serum of infected patients against these recombinant proteins using the very common ELISA test.

### REFERENCES

Dan J.M., J. Mateus, Y. Kato et al (2021) Immunological memory to SARS-CoV-2 assessed for up to eight months after infection. Science eabf4063
Ferretti A., T. Kula, Y. Wang et al., (2020) Unbiased screens show CD8+ T cells of COVID-19 patients recognize shared epitopes in SARS-CoV-2 that largely reside outside the spike protein. Immunity, 53: 1095-1107
Gallou C., A. Rougeot et al. (2016) A general strategynto optimize immunogenicity of HLA-B*0702 restricted cryptic peptides from tumor associated antigens: design of universal neo-antigen like tumor vaccine for HLA-B*0702 positive patients. Oncotarget, 37: 59417-428
Grifoni A., D. Weiskopf, S.I. Ramirez et al. (2020) Targets of T cell responses to SARS-CoV-2 coronavirus in humans with COVID-19 disease and unexposed individuals. Cell. 181:1489-1501
Le Bert N., A. Tan, K. Kunasegaran et al (2020) SARS-CoV-2-specific T cell immunity in cases of COVID-19 and SARS, and uninfected controls. Nature, 584: 457-462
Poland G.A., I.G. Ossyannikovan R.B. Kennedy. (2020) SARS-Cov-2 immunity: review and applications to phase 3 vaccine candidates. The Lancet, 396: 1595-1606
Ramasamy M., A. Minassian, K. Ewer et al. (2020) Safety and immunogenicity of ChAdOx1 nCov-19 vaccine administered in a prime-boost regimen in young and old adults (COV002): a single-blind, randomized, controlled phase 2/3 trial. The Lancet, 396: 1979-1993
Sahin U., A. Muik, E. Derhovannesian et al.(2000) COVID-19 vaccine BNT-162b1 elicits human antibody and Th1 T-cell responses. Nature 586:594-602
Scardino A., D.A. Gross et al., (2002) HER-2/neu and hTERT cryptic epitopes as novel targets for broad spectrum tumor immunotherapy. J. Immunol., 168: 5900-06
Schwarzkopf S., A. Krawczyk, D. Knop et al (2021) cellular immunity in COVID-19 convalescents with PCR-confirmed infection but with undetectable SARS-CoV-2-specific IgG. Emerging Inf. Dis., 27: 122-129
Sette A. and S. Crotty (2021) Adaptive Immunity to SARS-Cov-2 and COVID-19. Cell, 184: 1-20
Tarke A., J. Sidney, N. Methot et al. (2021) Negligeable impact of SARS-CoV-2 variants on CD4+ and CD8+ T cell reactivity in COVID-19 exposed donors and vaccinees. bioRxiv. Cell Rep. Med., 2: 100204
Tourdot, S., A. Scardino, et al. (2000). "A general strategy to enhance immunogenicity of low-affinity HLA-A2. 1-associated peptides: implication in the identification of cryptic tumor epitopes." Eur J Immunol 30(12): 3411-21.
Walsh E., R. Frenck, A.R. Falsey et al., (2020) Safety and immunogenicity of two RNA-based Covid-19 vaccine candidates. N. Eng. J. Med., 383: 2439-2450
Widge A.T., N.G. Rouphael, L.A. Jackcon et al., (2021) Durability of responses after SARS-CoV-2 mRNA-1273 vaccination. N. Engl. J. Med. 384:80-82
Zuo J., A. Dowell, H. Pearce et al. (2020) Robust SARS-CoV-2 specific T cell immunity is maintained at 6 months following primary infection. bioRxiv.

## Claims

1. A modified protein derived from a spike (S) protein of SARS-CoV2, **characterized in that** it comprises at least 4, preferably at least 5, more preferably at least 6 modifications selected from the group consisting of: L24A, K77Y, T109Y, D138Y, M153T, E169R, Q173Y, I197T, I714Y, I714A, S721Y, E725K, L727Y, S810Y, S813Y, K814Y, L821Y, G838T, Q853Y, G857Y, V915Y, S937Y, V976Y, S982Y, R983Y, V1040T, H1048Y, A1056Y, V1060Y, A1087Y, I1183T, N1192Y, W1212A, M1214R, G1223Y and V1264K, with the proviso that:
- positions 169 and 173 are not simultaneously modified;
- positions 714 and 721 are not simultaneously modified;
- at most one of positions 721, 725 and 727 is modified;
- at most one of positions 810, 813 and 814 is modified;
- at most one of positions 813, 814 and 821 is modified;
- positions 853 and 857 are not simultaneously modified;
- at most one of positions 976, 982 and 983 is modified;
- positions 1048 and 1056 are not simultaneously modified;
- positions 1056 and 1060 are not simultaneously modified; and
- positions 1212 and 1214 are not simultaneously modified.

2. The modified S protein of claim 1, **characterized in that** it comprises at least 2 modifications selected from the group consisting of: D138Y, E169R, Q173Y, I197T, E725K, G838T, Q853Y and N1192Y.

3. The modified S protein of claim 2, **characterized in that** it further comprises at least 1 modification selected from the group consisting of: K77Y, M153T, L821Y, V976Y, R983Y, H1048Y and V1060Y.

4. The modified S protein of claim 2 or claim 3, **characterized in that** it further comprises at least 1 modification selected from the group consisting of: T109Y, I714Y, S721Y, S810Y, S813Y, K814Y, G857Y, V915Y, V1040T, A1056Y, A1087Y and I1183T.

5. The modified S protein of any one of claims 2 to 4, **characterized in that** it further comprises the modification I714A and/or the modification L727Y.

6. The modified S protein of any one of claims 2 to 5, **characterized in that** it further comprises at least 1 modification selected from the group consisting of: L24A, S937Y, S982Y, W1212A, M1214R, G1223Y and V1264K.

7. The modified S protein of any one of claims 2 to 6, **characterized in that** it comprises modifications selected from at least 2, preferably at least 3, more preferably at least 4 and even more preferably 5, 6 or 7 of the following groups:
(i) group A2: T109Y, S721Y, L821Y, G857Y, V915Y, S937Y, V976Y, R983Y, H1048Y, V1060Y, N1192Y and G1223Y;
(ii) group B7: L24A, I714A and W1212A;
(iii) group A1: M153T, I197T, G838T, V1040T and I1183T;
(iv) group A3: E725K and V1264K;
(v) group A24: E169R and M1214R;
(vi) group B35: D138Y, Q173Y, I714Y, L727Yand A1056Y; and
(vii) group CW7: K77Y, S810Y, S813Y, K814Y, Q853Y, S982Y and A1087Y.

8. A nucleic acid encoding the modified S protein of any one of claims 1 to 7.

9. The nucleic acid of claim 8, which is a DNA molecule.

10. The nucleic acid of claim 8, which is an mRNA molecule.

11. An immunogenic composition comprising a modified S protein according to any one of claims 1 to 8 or a nucleic acid according to any one of claims 8 to 10.

12. The immunogenic composition of claim 11, which further comprises a modified protein derived from a nucleocapside protein (N) of SARS-CoV-2 or a nucleic acid encoding such a modified N protein, wherein said modified N protein comprises at least 2, preferably at least 3, more preferably at least 4 modifications selected from the group consisting of Q9Y, A35Y, L45A, P80T, R88Y, D103K, S105Y, T148Y, N150Y, N150A, S190Y, S202Y, Q229K, L291K, Q303Y, G316Y, W330K, K374Y and K387Y, with the proviso that:
- positions 103 and 105 are not simultaneously modified; and
- positions 148 and 150 are not simultaneously modified.

13. The immunogenic composition of claim 12, **characterized in that** said N protein comprises at least 1, preferably 2 modifications selected from the group consisting of: Q9Y, P80T, G316Y and K387Y.

14. The immunogenic composition of claim 12 or claim 13, **characterized in that** said modified N protein comprises at least 1, preferably at least 2 modifications selected from the group consisting of: A35Y, D103K, T148Y, N150A, S190Y, S202Y, Q229K and K374Y.

15. The immunogenic composition of any one of claims claim 12 to 14, **characterized in that** said modified N protein comprises at least 1 modification selected from the group consisting of: L45A, R88Y, S105Y, N150Y, L291K, Q303Y and W330K.

16. The immunogenic composition of any one of claims 12 to 15, **characterized in that** the S and N proteins comprise modifications selected from at least 2, preferably at least 3, more preferably at least 4 and even more preferably 5, 6 or 7 of the following groups:
(i) group A2: T109Y, S721Y, L821Y, G857Y, V915Y, S937Y, V976Y, R983Y, H1048Y, V1060Y, N1192Y and G1223Y in S protein and Q303Y and G316Y in N protein;
(ii) group B7: L24A, I714A and W1212A in S protein and L45A and N150A in N protein;
(iii) group A1: M153T, I197T, G838T, V1040T and I1183T in S protein and P80T in N protein;
(iv) the group A3: E725K and V1264K in S protein and Q229K, L291K, D103K and W330K in N protein;
(v) group A24: E169R and M1214R in S protein;
(vi) group B35: D138Y, Q173Y, I714Y, L727Yand A1056Y in S protein and S105Y and N150Y in N protein; and
(vii) group Cw7: K77Y, S810Y, S813Y, K814Y, Q853Y, S982Y and A1087Y in S protein and Q9Y, A35Y, R88Y, T148Y, S190Y, S202Y, K374Y and K387Y in N protein.


SEQUENCE LISTING

<110>  MENEZ, JEANNE
       KOSMATOPOULOS, KOSTANTINOS
       KINET, JEAN-PIERRE

<120>  Optimized vaccine to elicit a T cell immunity against SARS-CoV-2

<130>  B210048EPB

<160>  175

<170>  PatentIn version 3.5

<210>  1
<211>  1273
<212>  PRT
<213>  SARS-CoV-2

<400>  1

<210> 2
<211> 1273
<212> PRT
<213> artificial sequence

<220>
<223> modified S protein

<220>
<221> X
<222> (13)..(13)
<223> X = S or I

<220>
<221> X
<222> (18)..(18)
<223> X = L or F

<220>
<221> X
<222> (20)..(20)
<223> X = T or N

<220>
<221> X
<222> (24)..(24)
<223> X = L or A

<220>
<221> X
<222> (26)..(26)
<223> X = P or S; if X(24)=A then X(26)=P

<220>
<221>  X
<222>  (52)..(52)
<223>  X = Q or R

<220>
<221>  X
<222>  (77)..(77)
<223>  X = K or Y

<220>
<221>  X
<222>  (80)..(80)
<223>  X = D or A; if X(77) = Y then X(80) = D

<220>
<221>  X
<222>  (109)..(109)
<223>  X = T or Y

<220>
<221>  X
<222>  (138)..(138)
<223>  X = D or Y

<220>
<221>  X
<222>  (144)..(144)
<223>  X = Y or none; if X(138)=Y then X(144)=D

<220>
<221>  X
<222>  (152)..(152)
<223>  X = W or C; if X(153)=T then X(152)=W

<220>
<221>  X
<222>  (153)..(153)
<223>  X = M or T

<220>
<221>  X
<222>  (169)..(169)
<223>  X = E or R; if X (173) = Y then X (169) = E

<220>
<221>  X
<222>  (173)..(173)
<223>  X = Q or Y; if X (169) = R then X (173) = Q

<220>
<221>  X
<222>  (190)..(190)

<223>  X = R or S

<220>
<221>  X
<222>  (197)..(197)
<223>  X = I or T

<220>
<221>  X
<222>  (215)..(215)
<223>  X = D or G

<220>
<221>  X
<222>  (246)..(246)
<223>  X = R or I

<220>
<221>  X
<222>  (417)..(417)
<223>  X = K, N or T

<220>
<221>  X
<222>  (452)..(452)
<223>  X = L or R

<220>
<221>  X
<222>  (484)..(484)
<223>  X = E or K

<220>
<221>  X
<222>  (501)..(501)
<223>  X = N or Y

<220>
<221>  X
<222>  (570)..(570)
<223>  X = A or D

<220>
<221>  X
<222>  (614)..(614)
<223>  X = D or G

<220>
<221>  X
<222>  (655)..(655)
<223>  X = H or Y

<220>
<221>  X

<222>  (677)..(677)
<223>  X = Q or H

<220>
<221>  X
<222>  (681)..(681)
<223>  X = P or H

<220>
<221>  X
<222>  (701)..(701)
<223>  X = A or V

<220>
<221>  X
<222>  (714)..(714)
<223>  X = I, Y or A ; if X (721) = Y then X (714) = I

<220>
<221>  X
<222>  (716)..(716)
<223>  X = T or A; if X(714)=A or Y then X(716)=T

<220>
<221>  X
<222>  (721)..(721)
<223>  X = S or Y ; if X (714) = Y or A, or if X (725) = K, or if X
       (727) = Y, then X (721) = S

<220>
<221>  X
<222>  (725)..(725)
<223>  X = E or K ; if X (721) = Y, or if X (727) = Y, then X (725) = E

<220>
<221>  X
<222>  (727)..(727)
<223>  X = L or Y ; if X (721) = Y, or if X (725) = L, then X (727) = L

<220>
<221>  X
<222>  (810)..(810)
<223>  X = S or Y ; if X (813) = Y, or if X (814) = Y, then X (810) = S

<220>
<221>  X
<222>  (813)..(813)
<223>  X = S or Y ; if X (810) = Y, or if X (814) = Y, or if X (821) =
       Y, then X (813) = S

<220>
<221>  X
<222>  (814)..(814)
<223>  X = K or Y ; if X (810) = Y, or if X (813) = Y, or if X (821) =

Y, then X (814) = K

<220>
<221>  X
<222>  (821)..(821)
<223>  X = L or Y ; if X (813) = Y, or if X (814) = Y, then X (821) = L

<220>
<221>  X
<222>  (838)..(838)
<223>  X = G or T

<220>
<221>  X
<222>  (853)..(853)
<223>  X = Q or Y ; if X (857) = Y then X (853) = Q

<220>
<221>  X
<222>  (857)..(857)
<223>  X = G or Y ; if X (853) = Y then X (857) = G

<220>
<221>  X
<222>  (888)..(888)
<223>  X = F or L

<220>
<221>  X
<222>  (915)..(915)
<223>  X = V or Y

<220>
<221>  X
<222>  (937)..(937)
<223>  X = S or Y

<220>
<221>  X
<222>  (976)..(976)
<223>  X = V or Y ;  if X (982) = Y or if X (983) = Y then X (976) = V

<220>
<221>  X
<222>  (982)..(982)
<223>  X = S, A or Y ; if X (976) = Y then X (982) = S; if X (983) = Y
      then X (982) = S or A

<220>
<221>  X
<222>  (983)..(983)
<223>  X = R or Y ; if X (976) = Y or if X (982) = Y then X (983) = R

<220>

<221>  X
<222>  (1027)..(1027)
<223>  X = T or I

<220>
<221>  X
<222>  (1040)..(1040)
<223>  X = V or T

<220>
<221>  X
<222>  (1048)..(1048)
<223>  X = H or Y; if X(1056)=Y then X(1048)=H

<220>
<221>  X
<222>  (1056)..(1056)
<223>  X = A or Y ; if X(1048)=Y or X (1060) = Y then X (1056) = A

<220>
<221>  X
<222>  (1060)..(1060)
<223>  X = V or Y; if X (1056) = Y then X (1060) = V

<220>
<221>  X
<222>  (1087)..(1087)
<223>  X = A or Y

<220>
<221>  X
<222>  (1118)..(1118)
<223>  X =D or H

<220>
<221>  X
<222>  (1183)..(1183)
<223>  X = I or T

<220>
<221>  X
<222>  (1192)..(1192)
<223>  X = N or Y

<220>
<221>  X
<222>  (1212)..(1212)
<223>  X = W or A ; if X (1214) = R then X (1212) = W

<220>
<221>  X
<222>  (1214)..(1214)
<223>  X = M or R ; if X (1212) = A then X (1214) = M

<220>
<221>  X
<222>  (1223)..(1223)
<223>  X = G or Y

<220>
<221>  X
<222>  (1264)..(1264)
<223>  X = V or K

<400>  2

Met Phe Val Phe Leu Val Leu Leu Pro Leu Val Ser Xaa Gln Cys Val
1               5                   10                  15

Asn Xaa Thr Xaa Arg Thr Gln Xaa Pro Xaa Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Xaa Asp Leu Phe Leu Pro Phe Phe Ser Asn Val Thr Trp
    50                  55                  60

Phe His Ala Ile His Val Ser Gly Thr Asn Gly Thr Xaa Arg Phe Xaa
65                  70                  75                  80

Asn Pro Val Leu Pro Phe Asn Asp Gly Val Tyr Phe Ala Ser Thr Glu
                85                  90                  95

Lys Ser Asn Ile Ile Arg Gly Trp Ile Phe Gly Thr Xaa Leu Asp Ser
            100                 105                 110

Lys Thr Gln Ser Leu Leu Ile Val Asn Asn Ala Thr Asn Val Val Ile
        115                 120                 125

Lys Val Cys Glu Phe Gln Phe Cys Asn Xaa Pro Phe Leu Gly Val Xaa
    130                 135                 140

Tyr His Lys Asn Asn Lys Ser Xaa Xaa Glu Ser Glu Phe Arg Val Tyr
145                 150                 155                 160

Ser Ser Ala Asn Asn Cys Thr Phe Xaa Tyr Val Ser Xaa Pro Phe Leu
                165                 170                 175

Met Asp Leu Glu Gly Lys Gln Gly Asn Phe Lys Asn Leu Xaa Glu Phe
            180                 185                 190

Val Phe Lys Asn Xaa Asp Gly Tyr Phe Lys Ile Tyr Ser Lys His Thr
        195                 200                 205

Pro Ile Asn Leu Val Arg Xaa Leu Pro Gln Gly Phe Ser Ala Leu Glu
    210                 215                 220

Pro Leu Val Asp Leu Pro Ile Gly Ile Asn Ile Thr Arg Phe Gln Thr
225                 230                 235                 240

Leu Leu Ala Leu His Xaa Ser Tyr Leu Thr Pro Gly Asp Ser Ser Ser
                245                 250                 255

Gly Trp Thr Ala Gly Ala Ala Ala Tyr Tyr Val Gly Tyr Leu Gln Pro
            260                 265                 270

Arg Thr Phe Leu Leu Lys Tyr Asn Glu Asn Gly Thr Ile Thr Asp Ala
        275                 280                 285

Val Asp Cys Ala Leu Asp Pro Leu Ser Glu Thr Lys Cys Thr Leu Lys
    290                 295                 300

Ser Phe Thr Val Glu Lys Gly Ile Tyr Gln Thr Ser Asn Phe Arg Val
305                 310                 315                 320

Gln Pro Thr Glu Ser Ile Val Arg Phe Pro Asn Ile Thr Asn Leu Cys
                325                 330                 335

Pro Phe Gly Glu Val Phe Asn Ala Thr Arg Phe Ala Ser Val Tyr Ala
            340                 345                 350

Trp Asn Arg Lys Arg Ile Ser Asn Cys Val Ala Asp Tyr Ser Val Leu
        355                 360                 365

Tyr Asn Ser Ala Ser Phe Ser Thr Phe Lys Cys Tyr Gly Val Ser Pro
    370                 375                 380

Thr Lys Leu Asn Asp Leu Cys Phe Thr Asn Val Tyr Ala Asp Ser Phe
385                 390                 395                 400

Val Ile Arg Gly Asp Glu Val Arg Gln Ile Ala Pro Gly Gln Thr Gly
                405                 410                 415

Xaa Ile Ala Asp Tyr Asn Tyr Lys Leu Pro Asp Asp Phe Thr Gly Cys
            420                 425                 430

Val Ile Ala Trp Asn Ser Asn Asn Leu Asp Ser Lys Val Gly Gly Asn
        435                 440                 445

Tyr Asn Tyr Xaa Tyr Arg Leu Phe Arg Lys Ser Asn Leu Lys Pro Phe
    450                 455                 460

Glu Arg Asp Ile Ser Thr Glu Ile Tyr Gln Ala Gly Ser Thr Pro Cys
465                 470                 475                 480

Asn Gly Val Xaa Gly Phe Asn Cys Tyr Phe Pro Leu Gln Ser Tyr Gly
                485                 490                 495

Phe Gln Pro Thr Xaa Gly Val Gly Tyr Gln Pro Tyr Arg Val Val Val
            500                 505                 510

Leu Ser Phe Glu Leu Leu His Ala Pro Ala Thr Val Cys Gly Pro Lys
        515                 520                 525

Lys Ser Thr Asn Leu Val Lys Asn Lys Cys Val Asn Phe Asn Phe Asn
    530                 535                 540

Gly Leu Thr Gly Thr Gly Val Leu Thr Glu Ser Asn Lys Lys Phe Leu
545                 550                 555                 560

Pro Phe Gln Gln Phe Gly Arg Asp Ile Xaa Asp Thr Thr Asp Ala Val
                565                 570                 575

Arg Asp Pro Gln Thr Leu Glu Ile Leu Asp Ile Thr Pro Cys Ser Phe
            580                 585                 590

Gly Gly Val Ser Val Ile Thr Pro Gly Thr Asn Thr Ser Asn Gln Val
        595                 600                 605

Ala Val Leu Tyr Gln Xaa Val Asn Cys Thr Glu Val Pro Val Ala Ile
    610                 615                 620

His Ala Asp Gln Leu Thr Pro Thr Trp Arg Val Tyr Ser Thr Gly Ser
625                 630                 635                 640

Asn Val Phe Gln Thr Arg Ala Gly Cys Leu Ile Gly Ala Glu Xaa Val
                645                 650                 655

Asn Asn Ser Tyr Glu Cys Asp Ile Pro Ile Gly Ala Gly Ile Cys Ala
            660                 665                 670

Ser Tyr Gln Thr Xaa Thr Asn Ser Xaa Arg Arg Ala Arg Ser Val Ala
        675                 680                 685

Ser Gln Ser Ile Ile Ala Tyr Thr Met Ser Leu Gly Xaa Glu Asn Ser
    690                 695                 700

Val Ala Tyr Ser Asn Asn Ser Ile Ala Xaa Pro Xaa Asn Phe Thr Ile
705                 710                 715                 720

Xaa Val Thr Thr Xaa Ile Xaa Pro Val Ser Met Thr Lys Thr Ser Val
                725                 730                 735

Asp Cys Thr Met Tyr Ile Cys Gly Asp Ser Thr Glu Cys Ser Asn Leu
            740                 745                 750

Leu Leu Gln Tyr Gly Ser Phe Cys Thr Gln Leu Asn Arg Ala Leu Thr
        755                 760                 765

Gly Ile Ala Val Glu Gln Asp Lys Asn Thr Gln Glu Val Phe Ala Gln
    770                 775                 780

Val Lys Gln Ile Tyr Lys Thr Pro Pro Ile Lys Asp Phe Gly Gly Phe
785                 790                 795                 800

Asn Phe Ser Gln Ile Leu Pro Asp Pro Xaa Lys Pro Xaa Xaa Arg Ser
                805                 810                 815

Phe Ile Glu Asp Xaa Leu Phe Asn Lys Val Thr Leu Ala Asp Ala Gly
            820                 825                 830

Phe Ile Lys Gln Tyr Xaa Asp Cys Leu Gly Asp Ile Ala Ala Arg Asp
        835                 840                 845

Leu Ile Cys Ala Xaa Lys Phe Asn Xaa Leu Thr Val Leu Pro Pro Leu
    850                 855                 860

Leu Thr Asp Glu Met Ile Ala Gln Tyr Thr Ser Ala Leu Leu Ala Gly
865                 870                 875                 880

Thr Ile Thr Ser Gly Trp Thr Xaa Gly Ala Gly Ala Ala Leu Gln Ile
                885                 890                 895

Pro Phe Ala Met Gln Met Ala Tyr Arg Phe Asn Gly Ile Gly Val Thr
            900                 905                 910

Gln Asn Xaa Leu Tyr Glu Asn Gln Lys Leu Ile Ala Asn Gln Phe Asn
        915                 920                 925

Ser Ala Ile Gly Lys Ile Gln Asp Xaa Leu Ser Ser Thr Ala Ser Ala
    930                 935                 940

Leu Gly Lys Leu Gln Asp Val Val Asn Gln Asn Ala Gln Ala Leu Asn
945                 950                 955                 960

Thr Leu Val Lys Gln Leu Ser Ser Asn Phe Gly Ala Ile Ser Ser Xaa
                965                 970                 975

Leu Asn Asp Ile Leu Xaa Xaa Leu Asp Lys Val Glu Ala Glu Val Gln
            980                 985                 990

Ile Asp Arg Leu Ile Thr Gly Arg  Leu Gln Ser Leu Gln  Thr Tyr Val
        995                 1000                1005

Thr Gln  Gln Leu Ile Arg Ala  Ala Glu Ile Arg Ala  Ser Ala Asn
    1010                1015                1020

Leu Ala  Ala Xaa Lys Met Ser  Glu Cys Val Leu Gly  Gln Ser Lys
    1025                1030                1035

Arg Xaa Asp Phe Cys Gly Lys Gly Tyr Xaa Leu Met Ser Phe Pro
    1040                1045                1050

Gln Ser Xaa Pro His Gly Xaa Val Phe Leu His Val Thr Tyr Val
    1055                1060                1065

Pro Ala Gln Glu Lys Asn Phe Thr Thr Ala Pro Ala Ile Cys His
    1070                1075                1080

Asp Gly Lys Xaa His Phe Pro Arg Glu Gly Val Phe Val Ser Asn
    1085                1090                1095

Gly Thr His Trp Phe Val Thr Gln Arg Asn Phe Tyr Glu Pro Gln
    1100                1105                1110

Ile Ile Thr Thr Xaa Asn Thr Phe Val Ser Gly Asn Cys Asp Val
    1115                1120                1125

Val Ile Gly Ile Val Asn Asn Thr Val Tyr Asp Pro Leu Gln Pro
    1130                1135                1140

Glu Leu Asp Ser Phe Lys Glu Glu Leu Asp Lys Tyr Phe Lys Asn
    1145                1150                1155

His Thr Ser Pro Asp Val Asp Leu Gly Asp Ile Ser Gly Ile Asn
    1160                1165                1170

Ala Ser Val Val Asn Ile Gln Lys Glu Xaa Asp Arg Leu Asn Glu
    1175                1180                1185

Val Ala Lys Xaa Leu Asn Glu Ser Leu Ile Asp Leu Gln Glu Leu
    1190                1195                1200

Gly Lys Tyr Glu Gln Tyr Ile Lys Xaa Pro Xaa Tyr Ile Trp Leu
    1205                1210                1215

Gly Phe Ile Ala Xaa Leu Ile Ala Ile Val Met Val Thr Ile Met
    1220                1225                1230

Leu Cys  Cys Met Thr Ser Cys  Cys Ser Cys Leu Lys  Gly Cys Cys
    1235                    1240                    1245

Ser Cys  Gly Ser Cys Cys Lys  Phe Asp Glu Asp Asp  Ser Glu Pro
    1250                    1255                    1260

Xaa Leu  Lys Gly Val Lys Leu  His Tyr Thr
    1265                    1270

<210>  3
<211>  419
<212>  PRT
<213>  SARS-CoV-2

<400>  3

Met Ser Asp Asn Gly Pro Gln Asn Gln Arg Asn Ala Pro Arg Ile Thr
1               5                   10                  15

Phe Gly Gly Pro Ser Asp Ser Thr Gly Ser Asn Gln Asn Gly Glu Arg
            20                  25                  30

Ser Gly Ala Arg Ser Lys Gln Arg Arg Pro Gln Gly Leu Pro Asn Asn
        35                  40                  45

Thr Ala Ser Trp Phe Thr Ala Leu Thr Gln His Gly Lys Glu Asp Leu
    50                  55                  60

Lys Phe Pro Arg Gly Gln Gly Val Pro Ile Asn Thr Asn Ser Ser Pro
65                  70                  75                  80

Asp Asp Gln Ile Gly Tyr Tyr Arg Arg Ala Thr Arg Arg Ile Arg Gly
                85                  90                  95

Gly Asp Gly Lys Met Lys Asp Leu Ser Pro Arg Trp Tyr Phe Tyr Tyr
            100                 105                 110

Leu Gly Thr Gly Pro Glu Ala Gly Leu Pro Tyr Gly Ala Asn Lys Asp
        115                 120                 125

Gly Ile Ile Trp Val Ala Thr Glu Gly Ala Leu Asn Thr Pro Lys Asp
    130                 135                 140

His Ile Gly Thr Arg Asn Pro Ala Asn Asn Ala Ala Ile Val Leu Gln
145                 150                 155                 160

Leu Pro Gln Gly Thr Thr Leu Pro Lys Gly Phe Tyr Ala Glu Gly Ser
                165                 170                 175

Arg Gly Gly Ser Gln Ala Ser Ser Arg Ser Ser Ser Arg Ser Arg Asn
            180                 185                 190

Ser Ser Arg Asn Ser Thr Pro Gly Ser Ser Arg Gly Thr Ser Pro Ala
        195                 200                 205

Arg Met Ala Gly Asn Gly Gly Asp Ala Ala Leu Ala Leu Leu Leu Leu
    210                 215                 220

Asp Arg Leu Asn Gln Leu Glu Ser Lys Met Ser Gly Lys Gly Gln Gln
225                 230                 235                 240

Gln Gln Gly Gln Thr Val Thr Lys Lys Ser Ala Ala Glu Ala Ser Lys
                245                 250                 255

Lys Pro Arg Gln Lys Arg Thr Ala Thr Lys Ala Tyr Asn Val Thr Gln
            260                 265                 270

Ala Phe Gly Arg Arg Gly Pro Glu Gln Thr Gln Gly Asn Phe Gly Asp
        275                 280                 285

Gln Glu Leu Ile Arg Gln Gly Thr Asp Tyr Lys His Trp Pro Gln Ile
    290                 295                 300

Ala Gln Phe Ala Pro Ser Ala Ser Ala Phe Phe Gly Met Ser Arg Ile
305                 310                 315                 320

Gly Met Glu Val Thr Pro Ser Gly Thr Trp Leu Thr Tyr Thr Gly Ala
                325                 330                 335

Ile Lys Leu Asp Asp Lys Asp Pro Asn Phe Lys Asp Gln Val Ile Leu
            340                 345                 350

Leu Asn Lys His Ile Asp Ala Tyr Lys Thr Phe Pro Pro Thr Glu Pro

355                 360                 365

Lys Lys Asp Lys Lys Lys Lys Ala Asp Glu Thr Gln Ala Leu Pro Gln
    370                 375                 380

Arg Gln Lys Lys Gln Gln Thr Val Thr Leu Leu Pro Ala Ala Asp Leu
385                 390                 395                 400

Asp Asp Phe Ser Lys Gln Leu Gln Gln Ser Met Ser Ser Ala Asp Ser
                405                 410                 415

Thr Gln Ala

<210>  4
<211>  419
<212>  PRT
<213>  artificial sequece

<220>
<223>  modified N protein

<220>
<221>  X
<222>  (3)..(3)
<223>  X = D or L

<220>
<221>  X
<222>  (9)..(9)
<223>  X = Q or Y

<220>
<221>  X
<222>  (35)..(35)
<223>  X = A or Y

<220>
<221>  X
<222>  (45)..(45)
<223>  X = L or A

<220>
<221>  X
<222>  (80)..(80)
<223>  X = P, R or T

if X(79)=Y then X(80)=P

<220>
<221>  X
<222>  (88)..(88)
<223>  X = R or Y

<220>
<221>  X
<222>  (103)..(103)
<223>  X = D or K

if X(105)=Y then X(103)=D

<220>
<221>  X
<222>  (105)..(105)
<223>  X = S or Y

if X(103)=K then X(105)=S

<220>
<221>  X
<222>  (148)..(148)
<223>  X= T or Y; if X(150)=Y or A then X(148)=T

<220>
<221>  X
<222>  (150)..(150)
<223>  X= N, A or Y; if X(148)=Y then X(150)=N

<220>
<221>  X
<222>  (150)..(150)
<223>  X= N, A or Y; if X(148)=Y then X(150)=N

<220>
<221>  X
<222>  (190)..(190)
<223>  X = S or Y

<220>
<221>  X
<222>  (202)..(202)
<223>  X = S or Y

<220>
<221>  X
<222>  (229)..(229)
<223>  X = Q or K

<220>
<221>  X
<222>  (235)..(235)
<223>  X = S or F; if X(229) = K then X(235) = S

<220>
<221> X
<222> (291)..(291)
<223> X = L or K

<220>
<221> X
<222> (303)..(303)
<223> X = Q or Y

<220>
<221> X
<222> (316)..(316)
<223> X = G or Y

<220>
<221> X
<222> (330)..(330)
<223> X = W or K

<220>
<221> X
<222> (374)..(374)
<223> X = K or Y

<220>
<221> X
<222> (387)..(387)
<223> X = K or Y

<400> 4

Met Ser Xaa Asn Gly Pro Gln Asn Xaa Arg Asn Ala Pro Arg Ile Thr
1               5                   10                  15

Phe Gly Gly Pro Ser Asp Ser Thr Gly Ser Asn Gln Asn Gly Glu Arg
            20                  25                  30

Ser Gly Xaa Arg Ser Lys Gln Arg Arg Pro Gln Gly Xaa Pro Asn Asn
        35                  40                  45

Thr Ala Ser Trp Phe Thr Ala Leu Thr Gln His Gly Lys Glu Asp Leu
    50                  55                  60

Lys Phe Pro Arg Gly Gln Gly Val Pro Ile Asn Thr Asn Ser Ser Xaa
65                  70                  75                  80

Asp Asp Gln Ile Gly Tyr Tyr Xaa Arg Ala Thr Arg Arg Ile Arg Gly

                85                  90                  95

Gly Asp Gly Lys Met Lys Xaa Leu Xaa Pro Arg Trp Tyr Phe Tyr Tyr
            100                 105                 110

Leu Gly Thr Gly Pro Glu Ala Gly Leu Pro Tyr Gly Ala Asn Lys Asp
        115                 120                 125

Gly Ile Ile Trp Val Ala Thr Glu Gly Ala Leu Asn Thr Pro Lys Asp
    130                 135                 140

His Ile Gly Xaa Arg Xaa Pro Ala Asn Asn Ala Ala Ile Val Leu Gln
145                 150                 155                 160

Leu Pro Gln Gly Thr Thr Leu Pro Lys Gly Phe Tyr Ala Glu Gly Ser
                165                 170                 175

Arg Gly Gly Ser Gln Ala Ser Ser Arg Ser Ser Ser Arg Xaa Arg Asn
            180                 185                 190

Ser Ser Arg Asn Ser Thr Pro Gly Ser Xaa Arg Gly Thr Ser Pro Ala
        195                 200                 205

Arg Met Ala Gly Asn Gly Gly Asp Ala Ala Leu Ala Leu Leu Leu Leu
    210                 215                 220

Asp Arg Leu Asn Xaa Leu Glu Ser Lys Met Xaa Gly Lys Gly Gln Gln
225                 230                 235                 240

Gln Gln Gly Gln Thr Val Thr Lys Lys Ser Ala Ala Glu Ala Ser Lys
                245                 250                 255

Lys Pro Arg Gln Lys Arg Thr Ala Thr Lys Ala Tyr Asn Val Thr Gln
            260                 265                 270

Ala Phe Gly Arg Arg Gly Pro Glu Gln Thr Gln Gly Asn Phe Gly Asp
        275                 280                 285

Gln Glu Xaa Ile Arg Gln Gly Thr Asp Tyr Lys His Trp Pro Xaa Ile
    290                 295                 300

Ala Gln Phe Ala Pro Ser Ala Ser Ala Phe Phe Xaa Met Ser Arg Ile
305                 310                 315                 320

Gly Met Glu Val Thr Pro Ser Gly Thr Xaa Leu Thr Tyr Thr Gly Ala
                325                 330                 335

Ile Lys Leu Asp Asp Lys Asp Pro Asn Phe Lys Asp Gln Val Ile Leu
            340                 345                 350

Leu Asn Lys His Ile Asp Ala Tyr Lys Thr Phe Pro Pro Thr Glu Pro
        355                 360                 365

Lys Lys Asp Lys Lys Xaa Lys Ala Asp Glu Thr Gln Ala Leu Pro Gln
    370                 375                 380

Arg Gln Xaa Lys Gln Gln Thr Val Thr Leu Leu Pro Ala Ala Asp Leu
385                 390                 395                 400

Asp Asp Phe Ser Lys Gln Leu Gln Gln Ser Met Ser Ser Ala Asp Ser
                405                 410                 415

Thr Gln Ala

<210>  5
<211>  9
<212>  PRT
<213>  SARS-CoV-2

<400>  5

Thr Leu Asp Ser Lys Thr Gln Ser Leu
1               5

<210>  6
<211>  9
<212>  PRT
<213>  modified SARS-CoV-2

<400>  6

Tyr Leu Asp Ser Lys Thr Gln Ser Leu
1               5

<210>  7
<211>  9
<212>  PRT
<213>  SARS-CoV-2

<400>  7

Lys Ile Tyr Ser Lys His Thr Pro Ile
1               5

<210>  8
<211>  9
<212>  PRT
<213>  modified SARS-CoV-2

<400>  8

Tyr Ile Tyr Ser Lys His Thr Pro Ile
1               5

<210>  9
<211>  9
<212>  PRT
<213>  SARS-CoV-2

<400>  9

Lys Ile Ala Asp Tyr Asn Tyr Lys Leu
1               5

<210>  10
<211>  9
<212>  PRT
<213>  modified SARS-CoV-2

<400>  10

Tyr Ile Ala Asp Tyr Asn Tyr Lys Leu
1               5

<210>  11
<211>  9
<212>  PRT
<213>  SARS-CoV-2

<400>  11

Ser Val Thr Thr Glu Ile Leu Pro Val
1               5

<210>  12
<211>  9
<212>  PRT
<213>  modified SARS-CoV-2

<400>  12

Tyr Val Thr Thr Glu Ile Leu Pro Val
1               5

<210>  13
<211>  9
<212>  PRT
<213>  SARS-CoV-2

<400>  13

Leu Leu Phe Asn Lys Val Thr Leu Ala
1               5

<210>  14
<211>  9
<212>  PRT
<213>  SARS-CoV-2

<400>  14

Tyr Leu Phe Asn Lys Val Thr Leu Ala
1               5

<210>  15
<211>  9
<212>  PRT
<213>  SARS-CoV-2

<400>  15

Gly Leu Thr Val Leu Pro Pro Leu Leu
1               5

<210>  16
<211>  9
<212>  PRT
<213>  modified SARS-CoV-2

<400>  16

Tyr Leu Thr Val Leu Pro Pro Leu Leu

1               5


<210>  17
<211>  9
<212>  PRT
<213>  SARS-CoV-2

<400>  17

Val Leu Tyr Glu Asn Gln Lys Leu Ile
1               5


<210>  18
<211>  9
<212>  PRT
<213>  modified SARS-CoV-2

<400>  18

Tyr Leu Tyr Glu Asn Gln Lys Leu Ile
1               5


<210>  19
<211>  9
<212>  PRT
<213>  SARS-CoV-2

<400>  19

Ser Leu Ser Ser Thr Ala Ser Ala Leu
1               5


<210>  20
<211>  9
<212>  PRT
<213>  modified SARS-CoV-2

<400>  20

Tyr Leu Ser Ser Thr Ala Ser Ala Leu
1               5


<210>  21
<211>  9
<212>  PRT
<213>  SARS-CoV-2

<400>  21

Val Leu Asn Asp Ile Leu Ser Arg Leu
1               5

<210>  22
<211>  9
<212>  PRT
<213>  modified SARS-CoV-2

<400>  22

Tyr Leu Asn Asp Ile Leu Ser Arg Leu
1               5

<210>  23
<211>  9
<212>  PRT
<213>  SARS-CoV-2

<400>  23

Arg Leu Asp Lys Val Glu Ala Glu Val
1               5

<210>  24
<211>  9
<212>  PRT
<213>  modified SARS-CoV-2

<400>  24

Tyr Leu Asp Lys Val Glu Ala Glu Val
1               5

<210>  25
<211>  9
<212>  PRT
<213>  SARS-CoV-2

<400>  25

His Leu Met Ser Phe Pro Gln Ser Ala
1               5

<210>  26
<211>  9
<212>  PRT
<213>  modified SARS-CoV-2

<400>  26

Tyr Leu Met Ser Phe Pro Gln Ser Ala
1               5

<210>  27
<211>  9
<212>  PRT
<213>  SARS-CoV-2

<400>  27

Val Val Phe Leu His Val Thr Tyr Val
1               5

<210>  28
<211>  9
<212>  PRT
<213>  modified SARS-CoV-2

<400>  28

Tyr Val Phe Leu His Val Thr Tyr Val
1               5

<210>  29
<211>  9
<212>  PRT
<213>  SARS-CoV-2

<400>  29

Asn Leu Asn Glu Ser Leu Ile Asp Leu
1               5

<210>  30
<211>  9
<212>  PRT
<213>  modified SARS-CoV-2

<400>  30

Tyr Leu Asn Glu Ser Leu Ile Asp Leu
1               5

<210>  31
<211>  9
<212>  PRT
<213>  SARS-CoV-2

<400>  31

Gly Leu Ile Ala Ile Val Met Val Thr
1               5

<210>  32
<211>  9
<212>  PRT
<213>  modified SARS-CoV-2

<400>  32

Tyr Leu Ile Ala Ile Val Met Val Thr
1               5

<210>  33
<211>  9
<212>  PRT
<213>  SARS-CoV-2

<400>  33

Leu Pro Pro Ala Tyr Thr Asn Ser Phe
1               5

<210>  34
<211>  9
<212>  PRT
<213>  modified SARS-CoV-2

<400>  34

Ala Pro Pro Ala Tyr Thr Asn Ser Phe
1               5

<210>  35
<211>  9
<212>  PRT
<213>  SARS-CoV-2

<400>  35

Thr Pro Ile Asn Leu Val Arg Asp Leu
1               5

<210>  36
<211>  9
<212>  PRT
<213>  modified SARS-CoV-2

<400>  36

Ala Pro Ile Asn Leu Val Arg Asp Leu
1               5

<210>  37
<211>  9
<212>  PRT
<213>  SARS-CoV-2

<400>  37

Gly Pro Lys Lys Ser Thr Asn Leu Val
1               5

<210>  38
<211>  9
<212>  PRT
<213>  modified SARS-CoV-2

<400>  38

Ala Pro Lys Lys Ser Thr Asn Leu Val
1               5

<210>  39
<211>  9
<212>  PRT
<213>  SARS-CoV-2

<400>  39

Ile Pro Thr Asn Phe Thr Ile Ser Val
1               5

<210>  40
<211>  9
<212>  PRT
<213>  modified SARS-CoV-2

<400>  40

Ala Pro Thr Asn Phe Thr Ile Ser Val
1               5

<210>  41
<211>  9
<212>  PRT

<213>  SARS-CoV-2

<400>  41

Trp Pro Trp Tyr Ile Trp Leu Gly Phe
1               5

<210>  42
<211>  9
<212>  PRT
<213>  modified SARS-CoV-2

<400>  42

Ala Pro Trp Tyr Ile Trp Leu Gly Phe
1               5

<210>  43
<211>  9
<212>  PRT
<213>  SARS-CoV-2

<400>  43

Glu Tyr Val Ser Gln Pro Phe Leu Met
1               5

<210>  44
<211>  9
<212>  PRT
<213>  modified SARS-CoV-2

<400>  44

Arg Tyr Val Ser Gln Pro Phe Leu Met
1               5

<210>  45
<211>  9
<212>  PRT
<213>  SARS-CoV-2

<400>  45

Ala Tyr Tyr Val Gly Tyr Leu Gln Pro
1               5

<210>  46
<211>  9

<212>  PRT
<213>  modified SARS-CoV-2

<400>  46

Arg Tyr Tyr Val Gly Tyr Leu Gln Pro
1               5

<210>  47
<211>  9
<212>  PRT
<213>  SARS-CoV-2

<400>  47

Trp Tyr Ile Trp Leu Gly Phe Ile Ala
1               5

<210>  48
<211>  9
<212>  PRT
<213>  modified SARS-CoV-2

<400>  48

Arg Tyr Ile Trp Leu Gly Phe Ile Ala
1               5

<210>  49
<211>  9
<212>  PRT
<213>  SARS-CoV-2

<400>  49

Asp Pro Phe Leu Gly Val Tyr Tyr His
1               5

<210>  50
<211>  9
<212>  PRT
<213>  modified SARS-CoV-2

<400>  50

Tyr Pro Phe Leu Gly Val Tyr Tyr His
1               5

<210>  51

<211>  9
<212>  PRT
<213>  SARS-CoV-2

<400>  51

Gln Pro Phe Leu Met Asp Leu Glu Gly
1               5

<210>  52
<211>  9
<212>  PRT
<213>  modified SARS-CoV-2

<400>  52

Tyr Pro Phe Leu Met Asp Leu Glu Gly
1               5

<210>  53
<211>  9
<212>  PRT
<213>  SARS-CoV-2

<400>  53

Leu Pro Ile Gly Ile Asn Ile Thr Arg
1               5

<210>  54
<211>  9
<212>  PRT
<213>  modified SARS-CoV-2

<400>  54

Tyr Pro Ile Gly Ile Asn Ile Thr Arg
1               5

<210>  55
<211>  9
<212>  PRT
<213>  SARS-CoV-2

<400>  55

Gln Pro Arg Thr Phe Leu Leu Lys Tyr
1               5

<210>  56
<211>  9
<212>  PRT
<213>  modified SARS-CoV-2

<400>  56

Tyr Pro Arg Thr Phe Leu Leu Lys Tyr
1               5

<210>  57
<211>  9
<212>  PRT
<213>  SARS-CoV-2

<400>  57

Gln Pro Thr Glu Ser Ile Val Arg Phe
1               5

<210>  58
<211>  9
<212>  PRT
<213>  modified SARS-CoV-2

<400>  58

Tyr Pro Thr Glu Ser Ile Val Arg Phe
1               5

<210>  59
<211>  9
<212>  PRT
<213>  SARS-CoV-2

<400>  59

Cys Pro Phe Gly Glu Val Phe Asn Ala
1               5

<210>  60
<211>  9
<212>  PRT
<213>  modified SARS-CoV-2

<400>  60

Tyr Pro Phe Gly Glu Val Phe Asn Ala
1               5

<210> 61
<211> 9
<212> PRT
<213> SARS-CoV-2

<400> 61

Thr Pro Cys Asn Gly Val Glu Gly Phe
1               5

<210> 62
<211> 9
<212> PRT
<213> modified SARS-CoV-2

<400> 62

Tyr Pro Cys Asn Gly Val Glu Gly Phe
1               5

<210> 63
<211> 9
<212> PRT
<213> SARS-CoV-2

<400> 63

Ile Pro Ile Gly Ala Gly Ile Cys Ala
1               5

<210> 64
<211> 9
<212> PRT
<213> modified SARS-CoV-2

<400> 64

Tyr Pro Ile Gly Ala Gly Ile Cys Ala
1               5

<210> 65
<211> 9
<212> PRT
<213> SARS-CoV-2

<400> 65

Ile Pro Thr Asn Phe Thr Ile Ser Val
1               5

<210>  66
<211>  9
<212>  PRT
<213>  modified SARS-CoV-2

<400>  66

Tyr Pro Thr Asn Phe Thr Ile Ser Val
1               5



<210>  67
<211>  9
<212>  PRT
<213>  SARS-CoV-2

<400>  67

Leu Pro Val Ser Met Thr Lys Thr Ser
1               5



<210>  68
<211>  9
<212>  PRT
<213>  modified SARS-CoV-2

<400>  68

Tyr Pro Val Ser Met Thr Lys Thr Ser
1               5



<210>  69
<211>  9
<212>  PRT
<213>  SARS-CoV-2

<400>  69

Ala Pro His Gly Val Val Phe Leu His
1               5



<210>  70
<211>  9
<212>  PRT
<213>  modified SARS-CoV-2

<400>  70

Tyr Pro His Gly Val Val Phe Leu His

1                   5


<210>  71
<211>  9
<212>  PRT
<213>  SARS-CoV-2

<400>  71

Lys Arg Phe Asp Asn Pro Val Leu Pro
1                   5


<210>  72
<211>  9
<212>  PRT
<213>  modified SARS-CoV-2

<400>  72

Tyr Arg Phe Asp Asn Pro Val Leu Pro
1                   5


<210>  73
<211>  9
<212>  PRT
<213>  SARS-CoV-2

<400>  73

Ser Lys His Thr Pro Ile Asn Leu Val
1                   5


<210>  74
<211>  9
<212>  PRT
<213>  modified SARS-CoV-2

<400>  74

Tyr Lys His Thr Pro Ile Asn Leu Val
1                   5


<210>  75
<211>  9
<212>  PRT
<213>  SARS-CoV-2

<400>  75

Asn Arg Lys Arg Ile Ser Asn Cys Val
1               5

<210>  76
<211>  9
<212>  PRT
<213>  modified SARS-CoV-2

<400>  76

Tyr Arg Lys Arg Ile Ser Asn Cys Val
1               5

<210>  77
<211>  9
<212>  PRT
<213>  SARS-CoV-2

<400>  77

Ser Lys Pro Ser Lys Arg Ser Phe Ile
1               5

<210>  78
<211>  9
<212>  PRT
<213>  modified SARS-CoV-2

<400>  78

Tyr Lys Pro Ser Lys Arg Ser Phe Ile
1               5

<210>  79
<211>  9
<212>  PRT
<213>  SARS-CoV-2

<400>  79

Ser Lys Arg Ser Phe Ile Glu Asp Leu
1               5

<210>  80
<211>  9
<212>  PRT
<213>  modified SARS-CoV-2

<400>  80

Tyr Lys Arg Ser Phe Ile Glu Asp Leu
1               5

<210>  81
<211>  9
<212>  PRT
<213>  SARS-CoV-2

<400>  81

Lys Arg Ser Phe Ile Glu Asp Leu Leu
1               5

<210>  82
<211>  9
<212>  PRT
<213>  modified SARS-CoV-2

<400>  82

Tyr Arg Ser Phe Ile Glu Asp Leu Leu
1               5

<210>  83
<211>  9
<212>  PRT
<213>  SARS-CoV-2

<400>  83

Gln Lys Phe Asn Gly Leu Thr Val Leu
1               5

<210>  84
<211>  9
<212>  PRT
<213>  modified SARS-CoV-2

<400>  84

Tyr Lys Phe Asn Gly Leu Thr Val Leu
1               5

<210>  85
<211>  9
<212>  PRT
<213>  SARS-CoV-2

<400>  85

Ser Arg Leu Asp Lys Val Glu Ala Glu
1               5

<210>  86
<211>  9
<212>  PRT
<213>  modified SARS-CoV-2

<400>  86

Tyr Arg Leu Asp Lys Val Glu Ala Glu
1               5

<210>  87
<211>  9
<212>  PRT
<213>  SARS-CoV-2

<400>  87

Ala His Phe Pro Arg Glu Gly Val Phe
1               5

<210>  88
<211>  9
<212>  PRT
<213>  modified SARS-CoV-2

<400>  88

Tyr His Phe Pro Arg Glu Gly Val Phe
1               5

<210>  89
<211>  9
<212>  PRT
<213>  SARS-CoV-2

<400>  89

Trp Met Glu Ser Glu Phe Arg Val Tyr
1               5

<210>  90
<211>  9
<212>  PRT
<213>  modified SARS-CoV-2

<400>  90

Trp Thr Glu Ser Glu Phe Arg Val Tyr
1               5

<210>  91
<211>  9
<212>  PRT
<213>  SARS-CoV-2

<400>  91

Asn Ile Asp Gly Tyr Phe Lys Ile Tyr
1               5

<210>  92
<211>  9
<212>  PRT
<213>  modified SARS-CoV-2

<400>  92

Asn Thr Asp Gly Tyr Phe Lys Ile Tyr
1               5

<210>  93
<211>  9
<212>  PRT
<213>  SARS-CoV-2

<400>  93

Glu Arg Asp Ile Ser Thr Glu Ile Tyr
1               5

<210>  94
<211>  9
<212>  PRT
<213>  modified SARS-CoV-2

<400>  94

Glu Thr Asp Ile Ser Thr Glu Ile Tyr
1               5

<210>  95
<211>  9
<212>  PRT

<213>  SARS-CoV-2

<400>  95

Gly Val Glu Gly Phe Asn Cys Tyr Phe
1               5

<210>  96
<211>  9
<212>  PRT
<213>  modified SARS-CoV-2

<400>  96

Gly Thr Glu Gly Phe Asn Cys Tyr Phe
1               5

<210>  97
<211>  9
<212>  PRT
<213>  SARS-CoV-2

<400>  97

Ile Leu Asp Ile Thr Pro Cys Ser Phe
1               5

<210>  98
<211>  9
<212>  PRT
<213>  modified SARS-CoV-2

<400>  98

Ile Thr Asp Ile Thr Pro Cys Ser Phe
1               5

<210>  99
<211>  9
<212>  PRT
<213>  SARS-CoV-2

<400>  99

Tyr Gln Asp Val Asn Cys Thr Glu Val
1               5

<210>  100
<211>  9

<212>  PRT
<213>  modified SARS-CoV-2

<400>  100

Tyr Thr Asp Val Asn Cys Thr Glu Val
1               5



<210>  101
<211>  9
<212>  PRT
<213>  SARS-CoV-2

<400>  101

Gly Ala Glu His Val Asn Asn Ser Tyr
1               5



<210>  102
<211>  9
<212>  PRT
<213>  modified SARS-CoV-2

<400>  102

Gly Thr Glu His Val Asn Asn Ser Tyr
1               5



<210>  103
<211>  9
<212>  PRT
<213>  SARS-CoV-2

<400>  103

Tyr Gly Asp Cys Leu Gly Asp Ile Ala
1               5



<210>  104
<211>  9
<212>  PRT
<213>  modified SARS-CoV-2

<400>  104

Tyr Thr Asp Cys Leu Gly Asp Ile Ala
1               5



<210>  105

<211>  9
<212>  PRT
<213>  SARS-CoV-2

<400>  105

Arg Val Asp Phe Cys Gly Lys Gly Tyr
1               5



<210>  106
<211>  9
<212>  PRT
<213>  modified SARS-CoV-2

<400>  106

Arg Thr Asp Phe Cys Gly Lys Gly Tyr
1               5



<210>  107
<211>  9
<212>  PRT
<213>  SARS-CoV-2

<400>  107

Glu Ile Asp Arg Leu Asn Glu Val Ala
1               5



<210>  108
<211>  9
<212>  PRT
<213>  modified SARS-CoV-2

<400>  108

Glu Thr Asp Arg Leu Asn Glu Val Ala
1               5



<210>  109
<211>  9
<212>  PRT
<213>  SARS-CoV-2

<400>  109

Gly Ile Tyr Gln Thr Ser Asn Phe Arg
1               5

<210>  110
<211>  9
<212>  PRT
<213>  modified SARS-CoV-2

<400>  110

Lys Ile Tyr Gln Thr Ser Asn Phe Arg
1               5

<210>  111
<211>  9
<212>  PRT
<213>  SARS-CoV-2

<400>  111

Ser Val Tyr Ala Trp Asn Arg Lys Arg
1               5

<210>  112
<211>  9
<212>  PRT
<213>  modified SARS-CoV-2

<400>  112

Lys Val Tyr Ala Trp Asn Arg Lys Arg
1               5

<210>  113
<211>  9
<212>  PRT
<213>  SARS-CoV-2

<400>  113

Gln Ile Ala Pro Gly Gln Thr Gly Lys
1               5

<210>  114
<211>  9
<212>  PRT
<213>  modified SARS-CoV-2

<400>  114

Lys Ile Ala Pro Gly Gln Thr Gly Lys
1               5

<210> 115
<211> 9
<212> PRT
<213> SARS-CoV-2

<400> 115

Glu Ile Leu Pro Val Ser Met Thr Lys
1               5

<210> 116
<211> 9
<212> PRT
<213> modified SARS-CoV-2

<400> 116

Lys Ile Leu Pro Val Ser Met Thr Lys
1               5

<210> 117
<211> 9
<212> PRT
<213> SARS-CoV-2

<400> 117

Val Leu Lys Gly Val Lys Leu His Tyr
1               5

<210> 118
<211> 9
<212> PRT
<213> modified SARS-CoV-2

<400> 118

Lys Leu Lys Gly Val Lys Leu His Tyr
1               5

<210> 119
<211> 9
<212> PRT
<213> SARS-CoV-2

<400> 119

Gln Ile Ala Gln Phe Ala Pro Ser Ala
1               5

<210>  120
<211>  9
<212>  PRT
<213>  modified SARS-CoV-2

<400>  120

Tyr Ile Ala Gln Phe Ala Pro Ser Ala
1               5

<210>  121
<211>  9
<212>  PRT
<213>  SARS-CoV-2

<400>  121

Gly Met Ser Arg Ile Gly Met Glu Val
1               5

<210>  122
<211>  9
<212>  PRT
<213>  modified SARS-CoV-2

<400>  122

Tyr Met Ser Arg Ile Gly Met Glu Val
1               5

<210>  123
<211>  9
<212>  PRT
<213>  SARS-CoV-2

<400>  123

Leu Pro Asn Asn Thr Ala Ser Trp Phe
1               5

<210>  124
<211>  9
<212>  PRT
<213>  modified SARS-CoV-2

<400>  124

Ala Pro Asn Asn Thr Ala Ser Trp Phe

1                   5


<210>  125
<211>  9
<212>  PRT
<213>  SARS-CoV-2

<400>  125

Asn Pro Ala Asn Asn Ala Ala Ile Val
1                   5


<210>  126
<211>  9
<212>  PRT
<213>  modified SARS-CoV-2

<400>  126

Ala Pro Ala Asn Asn Ala Ala Ile Val
1                   5


<210>  127
<211>  9
<212>  PRT
<213>  SARS-CoV-2

<400>  127

Ser Pro Asp Asp Gln Ile Gly Tyr Tyr
1                   5


<210>  128
<211>  9
<212>  PRT
<213>  modified SARS-CoV-2

<400>  128

Ser Thr Asp Asp Gln Ile Gly Tyr Tyr
1                   5


<210>  129
<211>  9
<212>  PRT
<213>  SARS-CoV-2

<400>  129

Gln Leu Glu Ser Lys Met Ser Gly Lys
1               5

<210>  130
<211>  9
<212>  PRT
<213>  modified SARS-CoV-2

<400>  130

Lys Leu Glu Ser Lys Met Ser Gly Lys
1               5

<210>  131
<211>  9
<212>  PRT
<213>  SARS-CoV-2

<400>  131

Leu Ile Arg Gln Gly Thr Asp Tyr Lys
1               5

<210>  132
<211>  9
<212>  PRT
<213>  modified SARS-CoV-2

<400>  132

Lys Ile Arg Gln Gly Thr Asp Tyr Lys
1               5

<210>  133
<211>  9
<212>  PRT
<213>  SARS-CoV-2

<400>  133

Asp Leu Ser Pro Arg Trp Tyr Phe Tyr
1               5

<210>  134
<211>  9
<212>  PRT
<213>  modified SARS-CoV-2

<400>  134

Lys Leu Ser Pro Arg Trp Tyr Phe Tyr
1               5

<210>  135
<211>  9
<212>  PRT
<213>  SARS-CoV-2

<400>  135

Trp Leu Thr Tyr Thr Gly Ala Ile Lys
1               5

<210>  136
<211>  9
<212>  PRT
<213>  modified SARS-CoV-2

<400>  136

Lys Leu Thr Tyr Thr Gly Ala Ile Lys
1               5

<210>  137
<211>  9
<212>  PRT
<213>  SARS-CoV-2

<400>  137

Ser Pro Arg Trp Tyr Phe Tyr Tyr Leu
1               5

<210>  138
<211>  9
<212>  PRT
<213>  modified SARS-CoV-2

<400>  138

Tyr Pro Arg Trp Tyr Phe Tyr Tyr Leu
1               5

<210>  139
<211>  9
<212>  PRT
<213>  SARS-CoV-2

<400>  139

Asn Pro Ala Asn Asn Ala Ala Ile Val
1               5

<210>  140
<211>  9
<212>  PRT
<213>  modified SARS-CoV-2

<400>  140

Tyr Pro Ala Asn Asn Ala Ala Ile Val
1               5

<210>  141
<211>  9
<212>  PRT
<213>  SARS-CoV-2

<400>  141

Gln Arg Asn Ala Pro Arg Ile Thr Phe
1               5

<210>  142
<211>  9
<212>  PRT
<213>  modified SARS-CoV-2

<400>  142

Tyr Arg Asn Ala Pro Arg Ile Thr Phe
1               5

<210>  143
<211>  9
<212>  PRT
<213>  SARS-CoV-2

<400>  143

Ala Arg Ser Lys Gln Arg Arg Pro Gln
1               5

<210>  144
<211>  9
<212>  PRT
<213>  modified SARS-CoV-2

<400>  144

Tyr Arg Ser Lys Gln Arg Arg Pro Gln
1               5

<210>  145
<211>  9
<212>  PRT
<213>  SARS-CoV-2

<400>  145

Arg Arg Ala Thr Arg Arg Ile Arg Gly
1               5

<210>  146
<211>  9
<212>  PRT
<213>  modified SARS-CoV-2

<400>  146

Tyr Arg Ala Thr Arg Arg Ile Arg Gly
1               5

<210>  147
<211>  9
<212>  PRT
<213>  SARS-CoV-2

<400>  147

Thr Arg Asn Pro Ala Asn Asn Ala Ala
1               5

<210>  148
<211>  9
<212>  PRT
<213>  modified SARS-CoV-2

<400>  148

Tyr Arg Asn Pro Ala Asn Asn Ala Ala
1               5

<210>  149
<211>  9
<212>  PRT

<213>  SARS-CoV-2

<400>  149

Ser Arg Asn Ser Ser Arg Asn Ser Thr
1               5

<210>  150
<211>  9
<212>  PRT
<213>  modified SARS-CoV-2

<400>  150

Tyr Arg Asn Ser Ser Arg Asn Ser Thr
1               5

<210>  151
<211>  9
<212>  PRT
<213>  SARS-CoV-2

<400>  151

Ser Arg Gly Thr Ser Pro Ala Arg Met
1               5

<210>  152
<211>  9
<212>  PRT
<213>  modified SARS-CoV-2

<400>  152

Tyr Arg Gly Thr Ser Pro Ala Arg Met
1               5

<210>  153
<211>  9
<212>  PRT
<213>  SARS-CoV-2

<400>  153

Lys Lys Ala Asp Glu Thr Gln Ala Leu
1               5

<210>  154
<211>  9

<212>  PRT
<213>  modified SARS-CoV-2

<400>  154

Tyr Lys Ala Asp Glu Thr Gln Ala Leu
1               5

<210>  155
<211>  9
<212>  PRT
<213>  SARS-CoV-2

<400>  155

Lys Lys Gln Gln Thr Val Thr Leu Leu
1               5

<210>  156
<211>  9
<212>  PRT
<213>  modified SARS-CoV-2

<400>  156

Tyr Lys Gln Gln Thr Val Thr Leu Leu
1               5

<210>  157
<211>  9
<212>  PRT
<213>  SARS-CoV-2

<400>  157

Tyr Leu Gln Pro Arg Thr Phe Leu Leu
1               5

<210>  158
<211>  9
<212>  PRT
<213>  SARS-CoV-2

<400>  158

Leu Leu Leu Asp Arg Leu Asn Gln Leu
1               5

<210>  159

<211>  9
<212>  PRT
<213>  SARS-CoV-2

<400>  159

Ile Tyr Gln Thr Ser Asn Phe Arg Val
1               5

<210>  160
<211>  9
<212>  PRT
<213>  SARS-CoV-2

<400>  160

Gln Tyr Ile Lys Trp Pro Trp Tyr Ile
1               5

<210>  161
<211>  9
<212>  PRT
<213>  SARS-CoV-2

<400>  161

Lys Val Phe Arg Ser Ser Val Leu His
1               5

<210>  162
<211>  9
<212>  PRT
<213>  SARS-CoV-2

<400>  162

Lys Cys Tyr Gly Val Ser Pro Thr Lys
1               5

<210>  163
<211>  9
<212>  PRT
<213>  SARS-CoV-2

<400>  163

Lys Thr Phe Pro Pro Thr Glu Pro Lys
1               5

<210>  164
<211>  10
<212>  PRT
<213>  SARS-CoV-2

<400>  164

Gln Pro Tyr Arg Val Val Val Leu Ser Phe
1               5                   10

<210>  165
<211>  9
<212>  PRT
<213>  SARS-CoV-2

<400>  165

Phe Pro Gln Ser Ala Pro His Gly Val
1               5

<210>  166
<211>  9
<212>  PRT
<213>  SARS-CoV-2

<400>  166

Ser Pro Arg Trp Tyr Phe Tyr Tyr Leu
1               5

<210>  167
<211>  9
<212>  PRT
<213>  SARS-CoV-2

<400>  167

Val Ala Ser Gln Ser Ile Ile Ala Tyr
1               5

<210>  168
<211>  9
<212>  PRT
<213>  SARS-CoV-2

<400>  168

Lys Ala Tyr Asn Val Thr Gln Ala Phe
1               5

<210>  169
<211>  9
<212>  PRT
<213>  SARS-CoV-2

<400>  169

Ser Pro Asp Asp Gln Ile Gly Tyr Tyr
1               5

<210>  170
<211>  9
<212>  PRT
<213>  SARS-CoV-2

<400>  170

Phe Ala Pro Ser Ala Ser Ala Phe Phe
1               5

<210>  171
<211>  9
<212>  PRT
<213>  SARS-CoV-2

<400>  171

Thr Pro Ser Gly Thr Trp Leu Thr Tyr
1               5

<210>  172
<211>  9
<212>  PRT
<213>  SARS-CoV-2

<400>  172

Leu Pro Ala Ala Asp Leu Asp Asp Phe
1               5

<210>  173
<211>  9
<212>  PRT
<213>  SARS-CoV-2

<400>  173

Lys Thr Phe Pro Pro Thr Glu Pro Lys
1               5

<210>  174
<211>  10
<212>  PRT
<213>  SARS-CoV-2

<400>  174

Ala Thr Glu Gly Ala Leu Asn Thr Pro Lys
1               5                   10


<210>  175
<211>  9
<212>  PRT
<213>  SARS-CoV-2

<400>  175

Ala Ser Ala Phe Phe Gly Met Ser Arg
1               5